# EUROPEAN PATENT APPLICATION

(11) **EP 4 292 646 A1**
(43) Date of publication of application: **20.12.2023**
(21) Application number: 22752147.3
(22) Date of filing: 27.01.2022
(51) Int. Cl.: A61N 5/10

(54) **NEUTRON-CAPTURE THERAPY SYSTEM**

(30) Priority: 09.02.2021 CN 202110175204
(71) Applicant: Neuboron Therapy System Ltd., Xiamen, Fujian 361026 (CN)
(72) Inventor: LIU, Yuan-Hao, Jiangsu 211112 (CN); LU, Wei-Hua, Jiangsu 211112 (CN); GONG, Qiu-Ping, Jiangsu 211112 (CN); XU, Hao-Lei, Jiangsu 211112 (CN)
(74) Representative: Gunzelmann, Rainer
(86) International application number: PCT/CN2022/074192
(87) International publication number: WO 2022/170986

(57) **Abstract**

A neutron-capture therapy system (100), which is operated on the basis of an accelerator so as to be safer and more reliable, has a more compact structure and a reasonable layout, and may be applied to therapy places such as hospitals. The neutron-capture therapy system (100) comprises a charged particle beam generation unit (11), a beam transmission unit (12) and a neutron beam generation unit (13); the charged particle beam generation unit (11) comprises an ion source (111) and an accelerator (112), and the accelerator (112) accelerates charged particles generated by the ion source (111), so as to obtain a charged particle beam of the required energy; the neutron beam generation unit (13) comprises a target, a beam shaping body (131) and a collimator (132), the charged particle beam generated by the accelerator (112) irradiates onto the target through the beam transmission unit (12) and acts with the target to generate neutrons, and the generated neutrons sequentially pass through the beam shaping body (131) and the collimator (132) to form a neutron beam for therapy; and the neutron-capture therapy system (100) is integrally accommodated in a building constructed by concrete and comprises an irradiation room (101), an accelerator chamber (102) and a beam transmission chamber (103), and the neutron beam generation unit (13) is at least partially accommodated in a partition wall of the irradiation chamber(101) and the beam transmission chamber(103).

## Description

### TECHNICAL FIELD

The invention relates to a radiation ray irradiation system, and in particular to a neutron capture therapy system.

### BACKGROUND

With the development of atomics, radioactive ray therapy, such as cobalt sixty, a linear accelerator, an electron beam, or the like, has become one of the major means to treat cancers. However, traditional photon or electron therapy is restricted by physical conditions of radioactive rays themselves, and thus will also harm a large number of normal tissues on a beam path while killing tumor cells. Furthermore, owing to different levels of sensitivity of tumor cells to radioactive rays, traditional radiotherapy usually has poor treatment effect on malignant tumors (for example, glioblastoma multiforme and melanoma) with radio resistance.

In order to reduce radiation injury to normal tissues around tumors, a target therapy concept in chemotherapy is applied to radioactive ray therapy. With respect to tumor cells with high radio resistance, irradiation sources with high relative biological effectiveness (RBE), such as proton therapy, heavy particle therapy, neutron capture therapy, or the like, are also developed actively now Here neutron capture therapy combines the abovementioned two concepts, for example boron neutron capture therapy (BNCT), provides a better cancer treatment choice than traditional radioactive rays, by specific aggregation of boron-containing drugs in tumor cells in combination with precise neutron beam regulation and control.

Previous neutron capture therapy systems are mostly based on reactors, nuclear reactors themselves are expensive and limited in use, and also have unsafe factors, complex facilities, and is difficult to be used in medical applications. Therefore, it is necessary to propose a new technical solution to solve the above problems.

### SUMMARY

In order to solve the above problems, one aspect of the invention provides a neutron capture therapy system, including a charged particle beam generation part, a beam transmission part and a neutron beam generation part. The charged particle beam generation part includes an ion source and an accelerator, the ion source is configured to generate charged particles, the accelerator is configured to accelerate the charged particles generated by the ion source to obtain a charged particle beam with a required energy. The neutron beam generation part includes a target, a beam shaping body and a collimator, the target is arranged between the beam transmission part and the beam shaping body, the charged particle beam generated by the accelerator is irradiated onto the target through the beam transmission part and acts with the target to generate neutrons, and the generated neutrons sequentially pass through the beam shaping body and the collimator to form a therapeutic neutron beam. The neutron capture therapy system is entirely accommodated in a building made of concrete and includes an irradiation chamber, an accelerator chamber and a beam transmission chamber. An irradiated body injected with a medicament is subjected to irradiation treatment by the therapeutic neutron beam in the irradiation chamber. The accelerator chamber at least partially accommodates the charged particle beam generation part. The beam transmission chamber at least partially accommodates the beam transmission part, and the neutron beam generation part is at least partially accommodated in a partition wall between the irradiation chamber and the beam transmission chamber. The neutron capture therapy system is operated based on the accelerator, thus it is safer and more reliable, has a more compact structure and a reasonable layout, and may be applied to treatment sites such as hospitals or the like.

Preferably, the neutron capture therapy system may further include a medicament control chamber and a medicament injection device. The medicament injection device is configured to inject the medicament into the irradiated body during the irradiation treatment, and includes a medicament passage assembly, a medicament accommodation mechanism and a medicament control mechanism. The medicament passage assembly is arranged between the medicament control chamber and the irradiation chamber, and the medicament accommodation mechanism and the medicament control mechanism are arranged in the medicament control chamber and control injection of the medicament of the irradiated body in the medicament control chamber. Operations in the irradiation chamber may be avoided, to improve safety and reliability, and meanwhile, neutron radiation rays in the irradiation chamber are prevented from affecting the medicament accommodation mechanism and the medicament control mechanism.

Further, the medicament passage assembly may include a medicament passage member and an accommodation member. The medicament passage member is configured to inject the medicament. The accommodation member is configured to at least partially accommodate the medicament passage member, arranged in the partition wall and forms a passage for the medicament passage member to pass through the partition wall.

Preferably, the neutron capture therapy system may further include a treatment table, a treatment table positioning device, and a shielding device of the treatment table positioning device. The shielding device of the treatment table positioning device may reduce or avoid radiation damage to the treatment table positioning device caused by neutrons and other radioactive rays generated by the neutron capture therapy system, thereby prolonging service life thereof.

Further, the treatment table positioning device may include a robotic arm configured to support and position the treatment table and including at least one arm part, and the shielding device includes a robotic arm sheath surrounding the arm part.

Even further, the robotic arm sheath may be provided with an anti-collision protection mechanism. Or, the treatment table positioning device may further include a linear shaft, the robotic arm is arranged between the linear shaft and the treatment table, the linear shaft includes a sliding rail fixed to the building and a support seat connected to the robotic arm, the support seat drives the treatment table and the robotic arm to slide along the sliding rail together, and the shielding device includes a sliding rail covering member. The sliding rail covering member may reduce leakage of radiation rays caused when the support seat slides along the sliding rail.

Preferably, a neutron shielding space may be formed in the building and formed in the beam transmission chamber or the irradiation chamber, the concrete is a boron-containing barite concrete, or a neutron shielding plate is arranged on a surface of the concrete to form the neutron shielding space. Since a large number of neutrons are generated in a neutron capture therapy process, especially in vicinity of the neutron beam generation part, the neutron shielding space is provided to avoid or reduce neutron leakage or radiation damage and radiation pollution to other indoor devices as much as possible.

Preferably, the building may be internally provided with a cable for operations of the neutron capture therapy system, or a tubular member for gas and liquid to pass through, or a rod-shaped member fixedly mounted in the building, or a support device supporting the cable or the tubular member. A material of the support device, the tubular member or the rod-shaped member is composed of at least one of C, H, O, N, Si, Al, Mg, Li, B, Mn, Cu, Zn, S, Ca or Ti element, in 90% (percentage in terms of weight) or more thereof. The tubular member, the fixing rod, the cable and the support device of the tubular member are provided, and a material with less secondary radiation generated after neutron irradiation is selected, which may reduce radiation damage and radiation pollution. Or, a periphery of the cable, the tubular member or the rod-shaped member is provided with an annular shielding device including an inner sleeve, an outer sleeve, and a shielding material arranged between the inner sleeve and the outer sleeve. The annular shielding device is provided, which may reduce radiation damage and radiation pollution of neutrons generated by the neutron capture therapy system to the cable, the tubular member and the fixing rod arranged in the building.

Preferably, the neutron capture therapy system may further include an auxiliary device at least partially arranged in the accelerator chamber or the beam transmission chamber, and the auxiliary device includes a cooling device, or an insulation gas inflation and recovery device, or an air compression device providing compressed air, or a vacuum pump providing a vacuum environment.

Further, a cooling medium of the cooling device may have hardness less than 60 mg/L. The cooling device is used to cool to-be-cooled components of the neutron capture therapy system, thereby prolonging service life of the device. The cooling medium of the cooling device is soft water, so that scales are not easily generated on a water pipe during cooling, thereby affecting heat exchange efficiency.

Even further, the cooling device may be configured to cool the ion source or the accelerator or the target, a cooling medium of the cooling device has hardness less than 17 mg/L, or the cooling medium of the cooling device is deionized water with a conductivity of 0.5-1.5 µS/cm. Or, the cooling device may include an external circulation device, an internal circulation device and a heat exchanger. The internal circulation device delivers a cooling medium to a to-be-cooled component of the neutron capture therapy system to absorb heat thereof, then delivers the cooling medium after heat absorption and temperature rise to the heat exchanger, to perform heat exchange with chilled water delivered to the heat exchanger by the external circulation device, and then delivers the cooling medium after temperature dropping to the to-be-cooled component again to absorb heat thereof. The external circulation device is capable of continuously providing the chilled water to the heat exchanger and recovering the chilled water after heat absorption and temperature rise.

Further, the accelerator may include an accelerator high-voltage power supply providing an acceleration energy and internally provided with an insulation gas, to avoid breakdown of electronic components inside the accelerator high-voltage power supply. The insulation gas inflation and recovery device provides the insulation gas for the accelerator high-voltage power supply or recovers the insulation gas from the accelerator high-voltage power supply. The insulation gas may be recovered when related devices are maintained, examined and repaired, to improve utilization rate of the insulation gas.

Even further, the insulation gas inflation and recovery device may include a gas source and a storage container. The gas source includes a container containing the insulation gas, and the storage container is connected to the gas source and the accelerator high-voltage power supply respectively.

A second aspect of the invention provides a neutron capture therapy system, including a charged particle beam generation part, a beam transmission part and a neutron beam generation part. The charged particle beam generation part generates a charged particle beam. The beam transmission part transmits the charged particle beam to the neutron beam generation part, and the neutron beam generation part generates a therapeutic neutron beam. The neutron capture therapy system is entirely accommodated in a building made of concrete. The building is internally provided with a cable for operations of the neutron capture therapy system, or a tubular member for gas and liquid to pass through, or a rod-shaped member fixedly mounted in the building. A periphery of the cable, the tubular member or the rod-shaped member is provided with an annular shielding device. The annular shielding device is provided, which may reduce radiation damage and radiation pollution of neutrons generated by the neutron capture therapy system to the cable, the tubular member and the fixing rod arranged in the building.

Preferably, the annular shielding device may include an inner sleeve, an outer sleeve, and a shielding material arranged between the inner sleeve and the outer sleeve.

Further, a material of the inner sleeve or the outer sleeve may be composed of at least one of C, H, O, N, Si, Al, Mg, Li, B, Mn, Cu, Zn, S, Ca or Ti element, in 90% (percentage in terms of weight) or more thereof.

Further, the material of inner sleeve or the outer sleeve may be PVC.

Further, the outer sleeve may be used as a neutron retarder, and the retarded neutron may be better absorbed by the shielding material.

Further, the shielding material may be composed of a neutron shielding material.

Further, the shielding material may be a boron-containing resin.

Furthermore, preferably, the tubular member may be a ventilation pipe or a firefighting pipe, and the rod-shaped member is a support rod or a screw rod.

Furthermore, preferably, the charged particle beam generation part may include an accelerator, the neutron beam generation part may include a target, a beam shaping body and a collimator, the target is arranged between the beam transmission part and the beam shaping body, the charged particle beam generated by the accelerator is irradiated onto the target through the beam transmission part and acts with the target to generate neutrons, and the generated neutrons sequentially pass through the beam shaping body and the collimator to form a therapeutic neutron beam.

Further, the beam shaping body may include a reflector, a retarder, a thermal neutron absorber, a radiation shielding body and a beam outlet. The retarder decelerates neutrons generated from the target to an epithermal neutron energy region. The reflector surrounds the retarder and guides deviated neutrons back to the retarder to improve intensity of an epithermal neutron beam. The thermal neutron absorber is configured to absorb thermal neutrons to avoid excessive dose acting on normal tissues at a superficial layer during treatment. The radiation shielding body surrounds the beam outlet and is arranged at the rear of the reflector, to shield leaked neutrons and photons, so as to reduce dose acting on normal tissues at a non-irradiation area. The collimator is arranged at the rear of the beam outlet, to converge the neutron beam.

A third aspect of the invention provides a neutron capture therapy system, including a charged particle beam generation part, a beam transmission part and a neutron beam generation part. The charged particle beam generation part generates a charged particle beam. The beam transmission part transmits the charged particle beam to the neutron beam generation part, and the neutron beam generation part generates a therapeutic neutron beam. The neutron capture therapy system is entirely accommodated in a building made of concrete, and includes an irradiation chamber and a medicament control chamber. An irradiated body injected with a medicament is subj ected to irradiation treatment by the neutron beam in the irradiation chamber. The irradiation chamber has a partition wall separated from the medicament control chamber. The neutron capture therapy system further includes a medicament injection device, and the medicament injection device includes a medicament passage assembly arranged between the medicament control chamber and the irradiation chamber. The medicament passage assembly includes a medicament passage member and an accommodation member. The medicament passage member is configured to inject the medicament. The accommodation member is configured to at least partially accommodate the medicament passage member, arranged in the partition wall and forms a passage for the medicament passage member to pass through the partition wall. The medicament injection device injects the medicament into the irradiated body in the irradiation chamber by the medicament passage member passing through the partition wall, thereby avoiding operations in the irradiation chamber and improving safety and reliability. On one hand, providing the accommodation member is convenient for the medicament passage member to pass through, and on the other hand, providing the accommodation member separates the concrete wall, preventing dust or the like from polluting the medicament passage member.

Preferably, the medicament injection device may be configured to inject the medicament into the irradiated body during the irradiation treatment.

Preferably, the medicament injection device may further include a medicament accommodation mechanism and a medicament control mechanism. The medicament accommodation mechanism and the medicament control mechanism are arranged in the medicament control chamber and control injection of the medicament of the irradiated body in the medicament control chamber. Neutron radiation rays in the irradiation chamber may be prevented from affecting the medicament accommodation mechanism and the medicament control mechanism. Further, the medicament passage member is connected to the medicament accommodation mechanism, and the medicament is injected into the irradiated body by the medicament control mechanism.

Preferably, the accommodation member may be arranged in a through hole of the partition wall in a thickness direction.

Further, a central axis of the through hole may intersect with both the ground and a plane perpendicular to the ground along the thickness direction of the partition wall, so that radiation leakage may be reduced.

Further, a distance from center of the through hole located on a first side wall of the partition wall facing the medicament control chamber to the ground is greater than a distance from center of the through hole located on a second side wall of the partition wall facing the irradiation chamber to the ground.

Further, there are two or more through holes, and when one of the through holes is blocked or encounters other problems, the remaining through holes are used.

Preferably, material of the accommodation member may be PVC, and a product after subjecting to neutron irradiation does not have radioactivity or has extremely low radioactivity, thereby reducing the generated secondary radiation.

Preferably, the medicament passage member is at least partially made of a neutron shielding material, and influence of neutron radiation rays of the irradiation chamber on a boron-containing drug in the madicament passage member may be reduced.

A fourth aspect of the invention provides a neutron capture therapy system, including a charged particle beam generation part, a beam transmission part and a neutron beam generation part. The charged particle beam generation part generates a charged particle beam. The beam transmission part transmits the charged particle beam to the neutron beam generation part, and the neutron beam generation part generates a therapeutic neutron beam. The neutron capture therapy system is entirely accommodated in a building made of concrete, and a neutron shielding space is formed in the building made of concrete. Since a large number of neutrons are generated in a neutron capture therapy process, especially in vicinity of the neutron beam generation part, the neutron shielding space is provided to avoid or reduce neutron leakage or radiation damage and radiation pollution to other indoor devices as much as possible.

Preferably, the neutron capture therapy system may include an irradiation chamber and a beam transmission chamber, the beam transmission chamber at least partially accommodates the beam transmission part, the neutron beam generation part is at least partially accommodated in a partition wall between the irradiation chamber and the beam transmission chamber, and the neutron shielding space is formed in the beam transmission chamber or the irradiation chamber.

Furthermore, preferably, a neutron shielding plate may be arranged on a surface of the concrete to form the neutron shielding space.

Further, the neutron shielding plate may be arranged on the surface of the concrete through a support assembly, one side of the support assembly is connected to the concrete, and the other side of the support assembly is connected to the neutron shielding plate.

Even further, the neutron shielding plate may be a boron-containing PE plate, material of the support assembly is an aluminum alloy, and the support assembly includes two L-shaped plates connected to each other.

Furthermore, preferably, the neutron capture therapy system may further include an auxiliary device, and a neutron shielding plate is arranged around the auxiliary device to form the neutron shielding space, thereby reducing radiation damage and radiation pollution of neutrons to the auxiliary device in a neutron capture therapy process.

Further, the charged particle beam generation part may include an ion source and an accelerator, the ion source is configured to generate charged particles, the accelerator is configured to accelerate the charged particles generated by the ion source to obtain a charged particle beam with a required energy. The neutron capture therapy system further includes an accelerator chamber and a beam transmission chamber. The accelerator chamber at least partially accommodates the charged particle beam generation part. The beam transmission chamber at least partially accommodates the beam transmission part, and the auxiliary device is at least partially arranged in the accelerator chamber or the beam transmission chamber.

Further, an auxiliary device compartment may be provided to accommodate or surround the auxiliary device, and the auxiliary device compartment is at least partially made of a support assembly and the neutron shielding plate fixed on the support assembly.

Even further, the auxiliary device compartment may include a door and a mobile mechanism thereof, and the mobile mechanism may open the door to allow an operator to enter interior of the auxiliary device compartment, to facilitate examination, repairing, or the like of the device.

Even further, the mobile mechanism may include a guide rail and a sliding rod, and the door may slide along the guide rail in a horizontal direction through the sliding rod.

Even further, the mobile mechanism may further include a lifting assembly and a pulley, the lifting assembly may lift the door in a vertical direction to place the pulley at the bottom of the door, and the door may slide in the horizontal direction by means of the pulley, which is more labor-saving.

A fifth aspect of the invention provides a neutron capture therapy system, including a charged particle beam generation part, a beam transmission part, a neutron beam generation part, a treatment table and a treatment table positioning device. The charged particle beam generation part generates a charged particle beam. The beam transmission part transmits the charged particle beam to the neutron beam generation part, and the neutron beam generation part generates a therapeutic neutron beam. The treatment table positioning device includes a robotic arm configured to support and position the treatment table and including at least one arm part, and the neutron capture therapy system further includes a shielding device of the treatment table positioning device, the shielding device includes a robotic arm sheath surrounding the arm part. The shielding device of the treatment table positioning device may reduce radiation damage to the treatment table positioning device caused by neutrons and other radioactive rays generated by the neutron capture therapy system, thereby prolonging service life thereof.

Preferably, material of the robotic arm sheath may be at least partially a neutron shielding material, to prevent the arm part and metal components, electronic components, or the like arranged in mechanisms of the arm part from being activated by neutrons to become failure or damaged. Further, the material of the robotic arm sheath is at least partially a boron-containing glass fiber resin composite material, the glass fiber composite material has a certain strength and is not easily activated by neutrons, and boron may absorb neutrons.

Furthermore, preferably, the treatment table positioning device may further include a linear shaft, the robotic arm is arranged between the linear shaft and the treatment table, connects the treatment table to the linear shaft, and enables the treatment table and the robotic arm to slide along the linear shaft together. Further, the neutron capture therapy system may include an irradiation chamber and a preparation chamber, the linear shaft is configured to be fixed to a sliding rail in the irradiation chamber or the preparation chamber and a support seat connected to the robotic arm and sliding along the sliding rail, the shielding device includes a sliding rail covering curtain moving together with the support seat and always covering an exposed part of the sliding rail.

Furthermore, preferably, the robotic arm sheath may include a first housing and a second housing fixedly connected together and surrounding the arm part. Further, material of each of the first housing and the second housing may be a boron-containing glass fiber resin composite material, the glass fiber composite material has a certain strength and is not easily activated by neutrons, and boron may absorb neutrons, to prevent the arm part and metal components, electronic components, or the like arranged in mechanisms of the arm part from being activated by neutrons to become failure or damaged.

Furthermore, preferably, the robotic arm sheath may include a first housing and a second housing fixedly connected together and surrounding the arm part, and a third housing and a fourth housing fixedly connected together and surrounding the first housing and the second housing. The treatment table positioning device further includes an anti-collision protection mechanism, and the anti-collision protection mechanism includes a sensor arranged between the first housing and the third housing and/or between the second housing and the fourth housing.

Further, material of each of the first housing and the second housing may be a boron-containing glass fiber resin composite material, material of each of the third housing and the fourth housing is a glass fiber resin composite material, and a housing of the sensor is made of an aluminum alloy; or, material of each of the third housing and the fourth housing is a boron-containing glass fiber resin composite material. The glass fiber composite material has a certain strength and is not easily activated by neutrons, and boron may absorb neutrons, to prevent the arm part and metal components, electronic components, or the like arranged in mechanisms of the arm part from being activated by neutrons to become failure or damaged.

Further, the third housing or the fourth housing may be provided with a through hole at a position corresponding to the sensor, and the through hole is used for power supply and communication cables of the sensor to pass through.

Further, the first housing and the second housing may be provided with an accommodation cavity accommodating the sensor, and the sensor is arranged in the accommodation cavity and mounted between the first housing and the third housing and/or between the second housing and the fourth housing in an interference manner.

Further, a gap may be provided between the first housing and the third housing and/or between the second housing and the fourth housing, and configured to mount the sensor therein or used for power supply and communication cables of the sensor to pass through.

Further, the anti-collision protection mechanism may further include a sensor control assembly and a human-machine interface (HMI), the sensor is a pressure sensor, converts pressure acting on the third housing or the fourth housing into a pressure signal and transmits the pressure signal to the sensor control assembly, and displays a numerical value thereof in the HMI. When the pressure signal received by the sensor exceeds a preset value, the pressure signal exceeding the preset value is preferentially transmitted to the sensor control assembly and is displayed in the HMI in an alarm manner.

Furthermore, preferably, the treatment table positioning device may further include an anti-collision protection mechanism, the anti-collision protection mechanism includes a sensor arranged on the robotic arm sheath or between the robotic arm sheath and the arm part.

Further, the anti-collision protection mechanism may further include a sensor control assembly and an HMI, a signal transmitted by the sensor is transmitted to the sensor control assembly and displayed on the HMI, and the sensor control assembly performs corresponding control according to the received signal.

Further, the treatment table positioning device may further include a driving mechanism, the neutron capture therapy system may further include a treatment table control device connected to the driving mechanism and controlling movement of the robotic arm by controlling the driving mechanism, and the sensor control assembly transmits the received signal to the treatment table control device to perform corresponding control.

A sixth aspect of the invention provides a neutron capture therapy system, including a charged particle beam generation part, a beam transmission part, a neutron beam generation part, a treatment table and a treatment table positioning device. The charged particle beam generation part generates a charged particle beam. The beam transmission part transmits the charged particle beam to the neutron beam generation part, and the neutron beam generation part generates a therapeutic neutron beam. The neutron capture therapy system is entirely accommodated in a building made of concrete. The treatment table positioning device includes a linear shaft and a robotic arm arranged between the linear shaft and the treatment table to support and position the treatment table, and the linear shaft includes a sliding rail fixed to the building and a support seat connected to the robotic arm, the support seat drives the treatment table and the robotic arm to slide along the sliding rail together. The neutron capture therapy system further includes a shielding device of the treatment table positioning device, and the shielding device includes a sliding rail covering member. The shielding device of the treatment table positioning device may reduce or avoid radiation damage to the treatment table positioning device caused by neutrons and other radioactive rays generated by the neutron capture therapy system, thereby prolonging service life thereof, and the sliding rail covering member may reduce leakage of radiation rays caused when the support seat slides along the sliding rail.

Preferably, material of the sliding rail covering member may include a neutron shielding material.

Furthermore, preferably, the neutron capture therapy system may include an irradiation chamber, an irradiated body is subj ected to irradiation treatment by the neutron beam in the irradiation chamber, and the sliding rail is fixed on a fixing surface of the irradiation chamber.

Further, the sliding rail covering member may move together with the support seat and always cover an exposed part of the sliding rail.

Preferably, the fixing surface may be provided with a neutron shielding plate, and the sliding rail covering member is arranged between the support seat and the neutron shielding plate.

Furthermore, preferably, the sliding rail covering member may include a first part and a second part, and each of the first part and the second part includes flat plates connected in sequence.

Further, the flat plates may be slidably or pivotally connected in sequence.

Further, the sliding rail covering member may be supported by a support member of the sliding rail covering member, and one end, close to the support seat along a sliding direction of the support seat, of each of the first part and the second part is fixedly connected to the support seat, and the other end of each of the first part and the second part is fixedly connected to the support member.

Further, material of the support member may be a material of which a product after subjecting to neutron irradiation does not have radioactivity or has low radioactivity, or a radioactive isotope generated after subjecting to neutron irradiation has a short half-life period, and the neutron shielding plate covers the support member. Or, the material of the support member may include a neutron shielding material matching with the support member.

A seventh aspect of the invention provides a neutron capture therapy system, including a charged particle beam generation part, a beam transmission part and a neutron beam generation part. The charged particle beam generation part generates a charged particle beam. The beam transmission part transmits the charged particle beam to the neutron beam generation part, and the neutron beam generation part generates a therapeutic neutron beam. The neutron capture therapy system is entirely accommodated in a building made of concrete. The building is internally provided with a cable for operations of the neutron capture therapy system, or a tubular member for gas and liquid to pass through, or a rod-shaped member fixedly mounted in the building, or a support device supporting the cable or the tubular member. A material of the support device, the tubular member or the rod-shaped member is composed of at least one of C, H, O, N, Si, Al, Mg, Li, B, Mn, Cu, Zn, S, Ca or Ti element, in 90% (percentage in terms of weight) or more thereof. The tubular member, the fixing rod, the cable and the support device of the tubular member are provided, and a material with less secondary radiation generated after neutron irradiation is selected, which may reduce radiation damage and radiation pollution.

Preferably, the material of the support device, the tubular member or the rod-shaped member may be an aluminum alloy or plastic or rubber.

Furthermore, preferably, the support device may include a threading pipe for the cable to pass through and supporting the cable, and the threading pipe extends along an extension direction of the cable and is at least partially closed in a circumferential direction around the extension direction of the cable.

Further, a cross-sectional shape of the threading pipe in a direction perpendicular to the extension direction of the cable may be circular, polygonal, V -shaped, < -shaped, 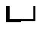 -shaped, or [-shaped.

Further, the threading pipe may be fixed on a wall or floor or ceiling in the building by connection members.

Further, the neutron capture therapy system may include an irradiation chamber, an accelerator chamber and a control chamber, an irradiated body is subjected to irradiation treatment by the neutron beam in the irradiation chamber, the accelerator chamber at least partially accommodates the charged particle beam generation part, the control chamber is configured to control the irradiation treatment of the neutron beam, and the threading pipe is arranged in the irradiation chamber, the accelerator chamber or the control chamber.

Furthermore, preferably, the support device may include a support frame configured to bear the tubular member or the cable and guide the tubular member or the cable.

Further, the support frame may have a bearing surface supporting the tubular member or the cable, and the support frame is fixed in a manner that the bearing surface is parallel to the ground or the bearing surface is perpendicular to the ground. Even further, the support frame may include side plates and transverse plates connected between the side plates at predetermined intervals, and the transverse plates form the bearing surface.

Further, the neutron capture therapy system may include an accelerator chamber and a beam transmission chamber, the accelerator chamber at least partially accommodates the charged particle beam generation part, the beam transmission chamber at least partially accommodates the beam transmission part, and the support frame is arranged in the accelerator chamber or the beam transmission chamber.

Furthermore, preferably, the charged particle beam generation part may include an accelerator, and the neutron beam generation part may include a target, a beam shaping body and a collimator, the target is arranged between the beam transmission part and the beam shaping body, a charged particle beam generated by the accelerator is irradiated onto the target through the beam transmission part and acts with the target to generate neutrons, and the generated neutrons sequentially pass through the beam shaping body and the collimator to form a therapeutic neutron beam.

Further, the beam shaping body may include a reflector, a retarder, a thermal neutron absorber, a radiation shielding body and a beam outlet. The retarder decelerates neutrons generated from the target to an epithermal neutron energy region. The reflector surrounds the retarder and guides deviated neutrons back to the retarder to improve intensity of an epithermal neutron beam. The thermal neutron absorber is configured to absorb thermal neutrons to avoid excessive dose acting on normal tissues at a superficial layer during treatment. The radiation shielding body surrounds the beam outlet and is arranged at the rear of the reflector, to shield leaked neutrons and photons, so as to reduce dose acting on normal tissues at a non-irradiation area. The collimator is arranged at the rear of the beam outlet, to converge the neutron beam.

An eighth aspect of the invention provides a neutron capture therapy system, including a charged particle beam generation part, a beam transmission part and a neutron beam generation part. The charged particle beam generation part generates a charged particle beam. The beam transmission part transmits the charged particle beam to the neutron beam generation part, and the neutron beam generation part generates a therapeutic neutron beam. The neutron capture therapy system further includes a cooling device, a cooling medium of the cooling device has hardness less than 60 mg/L. The cooling device is used to cool to-be-cooled components of the neutron capture therapy system, thereby prolonging service life of the device. The cooling medium of the cooling device is soft water, so that scales are not easily generated on a water pipe during cooling, thereby affecting heat exchange efficiency.

Preferably, the charged particle beam generation part may include an ion source and an accelerator, the ion source is configured to generate charged particles, the accelerator is configured to accelerate the charged particles generated by the ion source to obtain a charged particle beam with a required energy, and the cooling device is configured to cool the ion source or the accelerator.

Furthermore, preferably, the neutron beam generation part may include a target, the charged particle beam acts with the target to generate the neutron beam, and the cooling device is configured to cool the target, thereby prolonging service life of the target.

Further, the cooling medium of the cooling device may have hardness less than 17 mg/L, so that scales are not easily generated on a water pipe during cooling, thereby affecting heat exchange efficiency, especially in case that a heat exchange part uses a copper pipe; or, the cooling medium of the cooling device may have a conductivity less than 10 µS/cm, which may meet usage requirements under high-voltage conditions, and prevent generation of leakage current in a high-voltage environment and interference to generation of the neutron beam.

Further, the cooling medium of the cooling device may be deionized water with a conductivity of 0.5-1.5 µS/cm.

Preferably, the cooling device may include an external circulation device, an internal circulation device and a heat exchanger. The internal circulation device delivers a cooling medium to a to-be-cooled component of the neutron capture therapy system to absorb heat thereof, then delivers the cooling medium after heat absorption and temperature rise to the heat exchanger, to perform heat exchange with chilled water delivered to the heat exchanger by the external circulation device, and then delivers the cooling medium after temperature dropping to the to-be-cooled component again to absorb heat thereof. The external circulation device is capable of continuously providing the chilled water to the heat exchanger and recovering the chilled water after heat absorption and temperature rise.

Further, the external circulation device may include a cold source unit, a first pump, and a first control device controlling the cold source unit and the first pump. The external circulation device delivers the chilled water after heat absorption and temperature rise and coming out of the heat exchanger to the cold source unit so as to cool it, the cooled chilled water is pressurized by the first pump and delivered to the heat exchanger, and the first control device controls delivery of the chilled water.

Further, the internal circulation device may include a filter, a second pump, and a second control device controlling the filter and the second pump. One end of the internal circulation device is connected to the to-be-cooled component, and the other end of the internal circulation device is connected to the heat exchanger. The cooling medium absorbs heat of the to-be-cooled component and then is pressurized by the second pump and delivered to the heat exchanger, to perform heat exchange with the chilled water. The cooling medium after cooling and temperature dropping is filtered by the filter and then delivered into the to-be-cooled component for heat exchange, and the second control device controls delivery of the cooling medium.

Further, the internal circulation device may include a pressure stabilization loop or a cooling medium supplementary loop, the pressure stabilization loop and the cooling medium supplementary loop are controlled by the second control device, and the external circulation device include a chilled water supplementary loop controlled by the first control device.

A ninth aspect of the invention provides a neutron capture therapy system, including a charged particle beam generation part, a beam transmission part and a neutron beam generation part. The charged particle beam generation part generates a charged particle beam. The beam transmission part transmits the charged particle beam to the neutron beam generation part, and the neutron beam generation part generates a therapeutic neutron beam. The charged particle beam generation part includes an ion source and an accelerator, the ion source is configured to generate charged particles, the accelerator is configured to accelerate the charged particles generated by the ion source to obtain a charged particle beam with a required energy, and the accelerator includes an accelerator high-voltage power supply providing acceleration energy and internally provided with an insulation gas. The accelerator high-voltage power supply is internally provided with the insulation gas, to avoid breakdown of electronic components inside the accelerator high-voltage power supply.

Preferably, the neutron capture therapy system may further include an auxiliary device including an insulation gas inflation and recovery device. The insulation gas inflation and recovery device provides the insulation gas for the accelerator high-voltage power supply or recovers the insulation gas from the accelerator high-voltage power supply. The insulation gas may be recovered when related devices are maintained, examined and repaired, to improve utilization rate of the insulation gas.

Preferably, the insulation gas inflation and recovery device may include a gas source, and a storage container connected to the gas source and the accelerator high-voltage power supply respectively. The gas source includes a container containing the insulation gas.

Further, the insulation gas inflation and recovery device may further include a vacuum pump, and the vacuum pump is started before inflation, to vacuumize the storage container, pipes, components or the like of the insulation gas inflation and recovery device to discharge air in the device.

Further, the insulation gas inflation and recovery device may further include a compressor providing power for inflation and recovery (back inflation) processes.

Further, the insulation gas inflation and recovery device may further include a drying device arranged between the storage container and the accelerator high-voltage power supply, to remove most of water molecules in the recovered insulation gas to maintain the gas in a relatively dry state.

Further, the insulation gas inflation and recovery device may further include a filtering device arranged between the storage container and the accelerator high-voltage power supply, to remove oil, large-particle impurities or the like in the recovered insulation gas to maintain purity of the insulation gas.

Further, the insulation gas inflation and recovery device may further include a refrigeration device and a compression device arranged between the container of the gas source and the accelerator high-voltage power supply. When the insulation gas is inflated from the accelerator high-voltage power supply back into the container of the gas source, the refrigeration device converts the insulation gas into a liquid state, and the compression device compresses the insulation gas in a gaseous or liquid state, to fill it into the container of the gas source.

Preferably, the neutron beam generation part may include a target, a beam shaping body and a collimator, the target is arranged between the beam transmission part and the beam shaping body, a charged particle beam generated by the accelerator is irradiated onto the target through the beam transmission part and acts with the target to generate neutrons, and the generated neutrons sequentially pass through the beam shaping body and the collimator to form a therapeutic neutron beam.

Further, the beam shaping body may include a reflector, a retarder, a thermal neutron absorber, a radiation shielding body and a beam outlet. The retarder decelerates neutrons generated from the target to an epithermal neutron energy region. The reflector surrounds the retarder and guides deviated neutrons back to the retarder to improve intensity of an epithermal neutron beam. The thermal neutron absorber is configured to absorb thermal neutrons to avoid excessive dose acting on normal tissues at a superficial layer during treatment. The radiation shielding body surrounds the beam outlet and is arranged at the rear of the reflector, to shield leaked neutrons and photons, so as to reduce dose acting on normal tissues at a non-irradiation area. The collimator is arranged at the rear of the beam outlet, to converge the neutron beam.

The neutron capture therapy system according to the invention is operated based on the accelerator, thus it is safer and more reliable, has a more compact structure and a reasonable layout, and may be applied to treatment sites such as hospitals or the like.

### BRIEF DESCRIPTION OF THE DRAWINGS

FIG. 1 is a schematic structural diagram of a neutron capture therapy system according to an embodiment of the invention.
FIG. 2 is a schematic module diagram of a cooling device of a neutron capture therapy system according to an embodiment of the invention.
FIG. 3 is a schematic module diagram of an external circulation device of FIG. 2.
FIG. 4 is a schematic module diagram of an internal circulation device of FIG. 2.
FIG. 5 is a schematic module diagram of an insulation gas inflation and recovery device of a neutron capture therapy system according to an embodiment of the invention.
FIG. 6 is a schematic diagram of a planar layout of a neutron capture therapy system according to an embodiment of the invention.
FIG. 7 is a schematic diagram of a partition wall between a control chamber and an illumination chamber of FIG. 6.
FIG. 8A and FIG. 8B are schematic layout diagrams of a neutron shielding plate and a support assembly arranged at a side, facing a beam transmission chamber, of a partition wall between an irradiation chamber and a beam transmission chamber of a neutron capture therapy system according to an embodiment of the invention, here FIG. 8A is a schematic layout diagram of the neutron shielding plate, and FIG. 8B is a schematic layout diagram of the support assembly.
FIG. 9 is a schematic diagram of manners of fixing the neutron shielding plate and the support assembly of FIG. 8A and FIG. 8B.
FIG. 10 is a schematic diagram of an auxiliary device compartment arranged in a beam transmission chamber of a neutron capture therapy system according to an embodiment of the invention.
FIG. 11 is a schematic diagram of a treatment table positioning device of a neutron capture therapy system according to an embodiment of the invention.
FIG. 12 is a schematic diagram of FIG. 11 in another orientation.
FIG. 13 is a module diagram of a treatment table positioning device of a neutron capture therapy system and a control device thereof according to an embodiment of the invention.
FIG. 14 is a schematic diagram of an embodiment of a sliding rail covering member of the treatment table positioning device of FIG. 11.
FIG. 15 is a schematic diagram of another embodiment of a sliding rail covering member of the treatment table positioning device of FIG. 11.
FIG. 16 is a schematic diagram of an embodiment of a robotic arm sheath of the treatment table positioning device of FIG. 11.
FIG. 17 is a schematic layout diagram of a threading pipe and a support frame of a neutron capture therapy system according to an embodiment of the invention.
FIG. 18 is a schematic diagram of an annular shielding device of a neutron capture therapy system according to an embodiment of the invention.

### DETAILED DESCRIPTION

Embodiments of the invention will be further described in detail below with reference to the drawings, to enable those skilled in the art to implement the embodiments with reference to texts of the description.

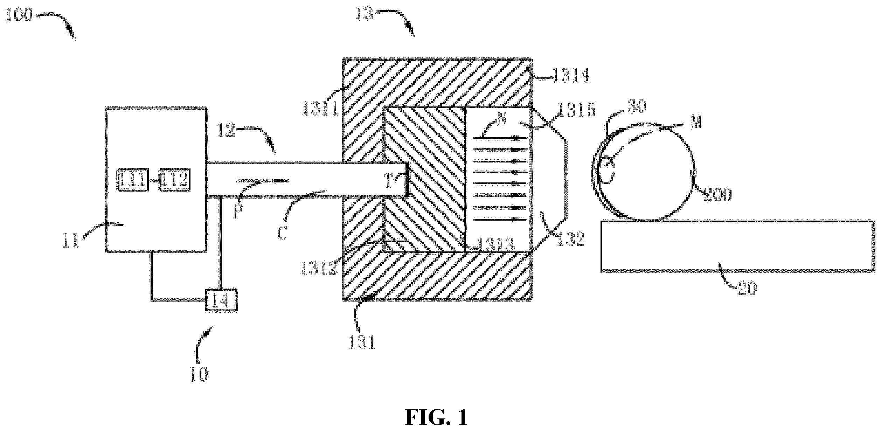

As shown in FIG. 1, a neutron capture therapy system in the embodiment is preferably a boron neutron capture therapy (BNCT) system 100 which is a device for performing cancer treatment by using boron neutron capture therapia. The boron neutron capture therapia performs cancer treatment by irradiating a neutron beam N onto an irradiated body 200 injected with boron (B-10). After the irradiated body 200 takes or is injected with a boron (B-10)-containing drug, the boron-containing drug is selectively aggregated in a tumor cell M, and then two heavily charged particles ⁴He and ⁷Li are produced by using a characteristic of the boron (B-10)-containing drug having a high capture section for a thermal neutron, and through ¹⁰B (n, α) ⁷Li neutron capture and a nuclear fission reaction. The two charged particles have an average energy of about 2.33 MeV, and have characteristics of high linear energy transfer (LET) and short range. LET and range of α particle are 150 keV/µm and 8 µm respectively, LET and range of the heavily charged particle ⁷Li are 175 keV/µm and 5 µm respectively, and the two particles have a total range approximately equivalent to a cell size, so that radiation injury to an organism may be limited to a cell level, and a purpose of locally killing the tumor cell may be achieved on premise of not inducing too large injury to normal tissues.

The BNCT system 100 includes a beam generation device 10 and a treatment table 20, and the beam generation device 10 includes a charged particle beam generation part 11, a beam transmission part 12 and a (first) neutron beam generation part 13. The charged particle beam generation part 11 generates a charged particle beam P such as a proton beam, the beam transmission part 12 transmits the charged particle beam P to the neutron beam generation part 13, and the neutron beam generation part 13 generates a therapeutic neutron beam N and irradiates the neutron beam N onto the irradiated body 200 on the treatment table 20.

The charged particle beam generation part 11 includes an ion source 111 and an accelerator 112, the ion source is configured to generate charged particles, such as H⁻, protons, deuterium cores, or the like, and the accelerator 112 is configured to accelerate the charged particles generated by the ion source 111 to obtain a charged particle beam P with a required energy, such as a proton beam.

The neutron beam generation part 13 includes a target T, a beam shaping body 131 and a collimator 132, the charged particle beam P generated by the accelerator 112 is irradiated onto the target T through the beam transmission part 12 and acts with the target T to generate neutrons, and the generated neutrons sequentially pass through the beam shaping body 131 and the collimator 132 to form a therapeutic neutron beam N and irradiate the neutron beam N onto the irradiated body 200 on the treatment table 20. Preferably, the target T is a metal target. An appropriate nuclear reaction is selected according to characteristics such as a desired neutron yield and energy, available energies of the accelerated charged particles, current, physical and chemical properties of the metal target, or the like. Nuclear reactions as commonly discussed include ⁷Li(p, n) ⁷Be and ⁹Be(p, n) ⁹B, both of which are endothermic reactions and have energy thresholds of 1.881 MeV and 2.055 MeV respectively. An ideal neutron source for BNCT is an epithermal neutron at a keV energy level, then theoretically, when protons with energies only slightly higher than the threshold are used to bombard a metallic lithium target, neutrons with relatively low energies may be generated for clinical application without too much retarding treatment. However, action sections of lithium (Li) and beryllium (Be) metallic targets with protons of threshold energies are not high, therefore protons with higher energies are usually selected to initiate a nuclear reaction, to generate a large enough neutron flux. An ideal target should have a high neutron yield, generate neutron energy with a distribution close to an epithermal neutron energy region (which will be described in detail below), not generate too much strong penetrating radiation, is safe, inexpensive, easy to operate and resistant to high temperature, or other characteristics. However, nuclear reactions meeting all requirements cannot be found actually. As well known to those skilled in the art, the target T may also be made of a metal material other than Li, Be, for example, formed of Ta or W, an alloy thereof, or the like. The accelerator 10 may be a linear accelerator, a cyclotron, a synchrotron, a synchrocyclotron.

The beam shaping body 131 may adjust beam quality of the neutron beam N generated by the charged particle beam P acting with the target T, and the collimator 132 is configured to converge the neutron beam N, so that the neutron beam N has high targeting during treatment. The beam shaping body 131 further includes a reflector 1311, a retarder 1312, a thermal neutron absorber 1313, a radiation shielding body 1314 and a beam outlet 1315. Neutrons generated by the charged particle beam P acting with the target T have a wide energy spectrum, therefore, except epithermal neutrons meeting treatment requirements, contents of other types of neutrons and photons need to be reduced as much as possible to avoid injury to an operator or the irradiated body. Therefore, neutrons coming out of the target T need to pass through the retarder 1312 to adjust energy (> 40 keV) of fast neutrons therein to the epithermal neutron energy region (0.5 eV to 40 keV) and reduce thermal neutrons (< 0.5 eV) as much as possible. The retarder 312 is made of a material with a large action section with fast neutrons and a small action section with thermal neutrons. In the embodiment, the retarder 312 is made of at least one of D₂O, AlF₃, Fluental, CaF₂, Li₂CO₃, MgF₂ or Al₂O₃. The reflector 1311 surrounds the retarder 1312, and reflects neutrons spreading all around through the retarder 1312 back to the neutron beam N to improve neutron utilization rate. The reflector 1311 is made of a material with strong neutron reflection ability. In the embodiment, the reflector 1311 is made of at least one of Pb or Ni. The thermal neutron absorber 1313 is arranged at the rear of the retarder 1312 and made of a material with a large action section with thermal neutrons. In the embodiment, the thermal neutron absorber 1313 is made of Li-6 and configured to absorb thermal neutrons passing through the retarder 1312 to reduce contents of thermal neutrons in the neutron beam N, avoiding excessive dose acting on normal tissues at a superficial layer during treatment. It may be understood that the thermal neutron absorber may also be integrated with the retarder, and material of the retarder contains Li-6. The radiation shielding body 1314 is configured to shield neutrons and photons leaked from parts other than the beam outlet 1315, and material of the radiation shielding body 1314 includes at least one of a photon shielding material or a neutron shielding material. In the embodiment, the material of the radiation shielding body 1314 includes lead (PB) used as the photon shielding material and polyethylene (PE) used as the neutron shielding material. It may be understood that the beam shaping body 131 may have other configurations as long as the desired epithermal neutron beam may be obtained for treatment. The collimator 132 is arranged at the rear of the beam outlet 1315, and the epithermal neutron beam coming out of the collimator 132 is irradiated onto the irradiated body 200, and is retarded as thermal neutrons after passing through normal tissues at a superficial layer, to reach the tumor cell M. It may be understood that the collimator 132 may also be omitted or replaced by other structures, and the neutron beam coming out of the beam outlet 1315 is directly irradiated onto the irradiated body 200. In the embodiment, a radiation shielding device 30 is further arranged between the irradiated body 200 and the beam outlet 1315 to shield irradiation of the beam coming out of the beam outlet 1315 to normal tissues of the irradiated body. It may be understood that the radiation shielding device 30 may not be provided, either. The target T is arranged between the beam transmission part 12 and the beam shaping body 131, and the beam transmission part 12 has a transmission pipe C configured to accelerate or transmit the charged particle beam P. In the embodiment, the transmission pipe C extends into the beam shaping body 131 along a direction of the charged particle beam P and sequentially passes through the reflector 1311 and the retarder 1312, and the target T is arranged in the retarder 1312 and located at an end of the transmission pipe C to obtain a better neutron beam quality. It may be understood that the target may be arranged in other ways, and may also be movable relative to the accelerator or the beam shaping body, to facilitate target replacement or make the charged particle beam act with the target uniformly.

The BNCT system 100 further includes an auxiliary device 14, and the auxiliary device 14 may include any auxiliary device providing preconditions for the charged particle beam generation part 11, the beam transmission part 12 and the neutron beam generation part 13 to operate. In an embodiment, the auxiliary device 14 includes a cooling device 141, an air compression device providing compressed air, an insulation gas inflation and recovery device 142, a vacuum pump 143 providing a vacuum environment, or the like, which is not specifically limited in the invention.

The cooling device 141 may be configured to cool to-be-cooled component CP such as the charged particle beam generation part 11, the target T, other auxiliary devices 14, or the like, thereby prolonging service life of the device. A cooling medium of the cooling device 141 may be soft water, so that scales are not easily generated on a water pipe during cooling, thereby affecting heat exchange efficiency, especially in case that a heat exchange part uses a copper pipe, for example, the cooling medium has hardness less than 60 mg/L. When the cooling device 141 is configured to cool the charged particle beam generation part 11 and the target T, the cooling medium must have an extremely low conductivity, for example, conductivity of the cooling medium is less than 10 µS/cm, to meet usage requirements under high-voltage conditions, and prevent generation of leakage current in a high-voltage environment and interference to generation of the neutron beam. In the embodiment, two sets of cooling devices are provided, one set of cooling devices uses soft water with hardness less than 17 mg/L, and the other set of cooling devices uses deionized water with a conductivity of 0.5-1.5 µS/cm. It may be understood that other types of cooling media may also be used.

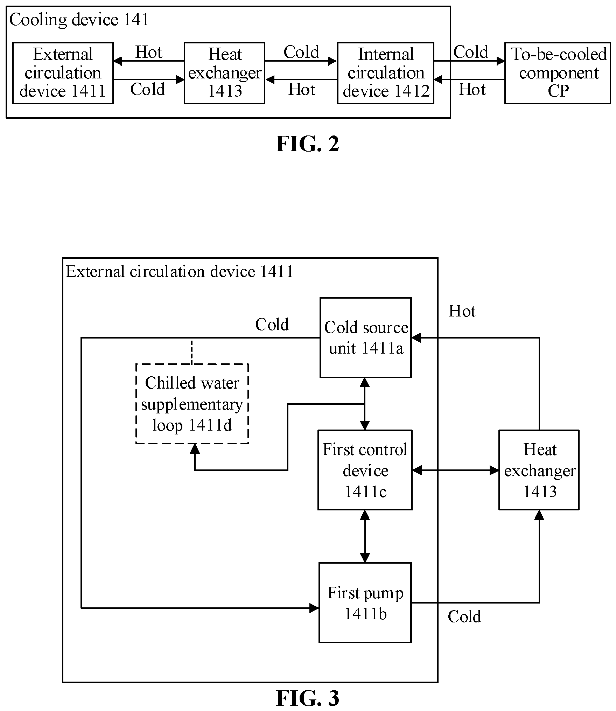

As shown in FIG. 2, the cooling device 141 includes an external circulation device 1411, an internal circulation device 1412 and a heat exchanger 1413. The internal circulation device 1412 delivers a cooling medium (such as soft water or deionized water) to the to-be-cooled component CP to absorb heat thereof, then delivers the cooling medium after heat absorption and temperature rise to the heat exchanger 1413, to perform heat exchange with chilled water delivered to the heat exchanger 1413 by the external circulation device 1411, and then delivers the cooling medium after temperature dropping to the to-be-cooled component CP again to absorb heat thereof, and the process is repeated as above. The external circulation device 1411 is capable of continuously providing the chilled water to the heat exchanger 1413 and recovering the chilled water after heat absorption and temperature rise. The external circulation device 1411 is arranged outdoors, that is, outside a building (which will be described in detail below) accommodating the BNCT system 100, to discharge heat to the atmosphere. In the embodiment, the external circulation device 1411 is arranged on the roof of the building. The internal circulation device 1412 and the heat exchanger 1413 are arranged indoors, that is, inside the building accommodating the BNCT system 100, to absorb heat of the to-be-cooled component CP. It may be understood that other arrangements may also be provided, for example, the heat exchanger is arranged outdoors.

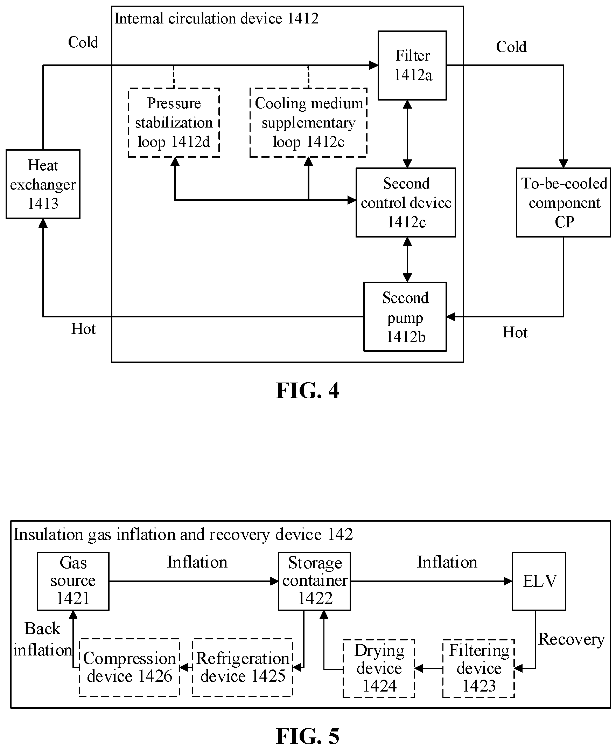

As shown in FIG. 3, the external circulation device 1411 may include a cold source unit 141 1a, a first pump 141 1b, a first control device 1411c controlling the cold source unit 1411a and the first pump 1411b, or the like. The chilled water after heat absorption and temperature rise and coming out of the heat exchanger 1413 is delivered to the cold source unit 1411a so as to be cooled, the cooled chilled water is pressurized by the first pump 1411b and delivered to the heat exchanger 1413, and the first control device 1411c controls delivery of the chilled water. As shown in FIG. 4, the internal circulation device 1412 may include a filter 1412a, a second pump 1412b, a second control device 1412c controlling the filter 1412a and the second pump 1412b, or the like. One end of the internal circulation device 1412 is connected to the to-be-cooled component CP, and the other end of the internal circulation device 1412 is connected to the heat exchanger 1413. The cooling medium absorbs heat of the to-be-cooled component CP at an end and then is pressurized by the second pump 1412b and delivered to the heat exchanger 1413, to perform heat exchange with the chilled water. The cooling medium after cooling and temperature dropping is filtered by the filter 1412a and then delivered into the to-be-cooled component CP for heat exchange, and the second control device 1412c controls delivery of the cooling medium. When the cooling medium uses deionized water, the cooling medium is affected by various factors during circulation, so that conductivity thereof is continuously increased, conductivity of the cooling medium is maintained through the filter to meet requirements. A conductivity sensor (not shown in the figure) may also be provided to detect conductivity of the cooling medium at an outlet of the filter 1412a, to ensure that requirements are met. In the embodiment, the heat exchanger 1413 is also controlled by the first control device 1411c, and it may be understood that the heat exchanger 1413 may also have a separate control device or may be controlled by the second control device 1412c.

The internal circulation device 1412 may further include a pressure stabilization loop 1412d controlled by the second control device 1412c. In an embodiment, the pressure stabilization loop 1412d may include a buffer tank, a nitrogen tank, a pressure sensor, or the like. Pressure in the nitrogen tank is detected by the pressure sensor, and nitrogen is supplemented to the buffer tank when the pressure is less than a set value, so that pressure is increased, positive pressure in the system is ensured, and air is prevented from entering the system. The external circulation device 1411 and the internal circulation device 1412 may further include a chilled water supplementary loop 1411d and a cooling medium supplementary loop 1412e respectively which are controlled by the first control device 1411c and the second control device 1412c respectively, an alarm prompt may occur when the chilled water/cooling medium is insufficient, and the chilled water/cooling medium is supplemented through the chilled water supplementary loop 1411d/the cooling medium supplementary loop 1412e. Each of the external circulation device 1411 and the internal circulation device 1412 may further include a temperature sensor, a regulation valve, a pressure sensor, or the like controlled by the first control device 1411c and the second control device 1412c. It may be understood that the cooling device 141 may also have other configurations.

The accelerator 112 includes an accelerator high-voltage power supply (ELV) 1121 providing an acceleration energy, and an insulation gas should be provided to the accelerator high-voltage power supply 1211 (for example, the insulation gas is arranged in a housing of the accelerator high-voltage power supply 1121), to prevent breakdown of electronic components inside the accelerator high-voltage power supply 1211. The insulation gas may be SF₆, and it may be understood that other insulation gases may also be used. The insulation gas inflation and recovery device 142 provides the insulation gas for the accelerator high-voltage power supply 1121 or recovers the insulation gas from the accelerator high-voltage power supply 1121. The insulation gas may be recovered when related devices are maintained, examined and repaired, to improve utilization rate of the insulation gas.

As shown in FIG. 5, the insulation gas inflation and recovery device 142 includes a gas source 1421 (for example, a steel cylinder containing SF₆) and a storage container 1422 connected to the gas source 1421 and the accelerator high-voltage power supply 1121 respectively. In an initial state, an insulation gas is accommodated in the container of the gas source 1421, then the insulation gas is inflated from the container of the gas source 1421 into the storage container 1422 and then inflated from the storage container 1422 into the ELV, so that the ELV may start to operate normally. When the ELV is required to be opened for maintenance, examination, repairing, or the like, the insulation gas is recovered from the ELV into the storage container 1422, and after maintenance, examination and repairing are completed, the insulation gas is inflated from the storage container 1422 into the ELV. When the storage container 1422, pipes, components, or the like of the insulation gas inflation and recovery device 142 need to be maintained or generate failure to be examined and repaired, the insulation gas may be inflated from the storage container 1422 back to the container of the gas source 1421, to return to the initial state, and after maintenance, examination and repairing are completed, the insulation gas is inflated again.

The insulation gas inflation and recovery device 142 may further include a filtering device 1423 and a drying device 1424 arranged between the storage container 1422 and the ELV. When the insulation gas is recovered from the ELV into the storage container 1422, the filtering device 1423 removes oil, large-particle impurities or the like in the recovered insulation gas to maintain purity of the insulation gas, and the drying device 1424 removes most of water molecules in the recovered insulation gas to maintain the gas in a relatively dry state. The filtering device 1423 may be a filtering screen, and the drying device 1424 may perform drying by electrical heating, or drying or filtering may be performed in other ways. In the embodiment, the insulation gas passes through the filtering device 1423 and then passes through the drying device 1424, it may be understood that the insulation gas may also be dried and then filtered, the filtering device 1423 may also be integrated with the drying device 1424, and a moisture detection component, or an oil detection component, or an impurity detection component may also be included.

The insulation gas inflation and recovery device 142 may further include a refrigeration device 1425 and a compression device 1426 arranged between the container of the gas source 1421 and the storage container 1422. When the insulation gas is inflated from the storage container 1422 back into the container of the gas source 1421, the refrigeration device 1425 converts the insulation gas into a liquid state, and the compression device 1426 compresses the insulation gas in a gaseous or liquid state, to fill it into the container of the gas source 1421. It may be understood that a sequence of the refrigeration device 1425 and the compression device 1426 is not limited, and the refrigeration device 1425 may also be integrated with the compression device 1426.

The insulation gas inflation and recovery device 142 may further include a vacuum pump, and the vacuum pump is started before inflation, to vacuumize the storage container 1422, pipes, components or the like of the insulation gas inflation and recovery device 142 to discharge air in the device. The accelerator high-voltage power supply 1121 may also be provided with a vacuum pump 143 which vacuumizes the ELV before inflation of the ELV and operation of the ELV, to discharge air. The insulation gas inflation and recovery device 142 may also include a compressor providing power for the above inflation and recovery (back inflation) processes. The insulation gas inflation and recovery device 142 may further include a valve, a vacuum degree detection component, a pressure detection component, or the like, to control the above inflation and recovery (back inflation) processes. It may be understood that the insulation gas inflation and recovery device 142 may also have other configurations.

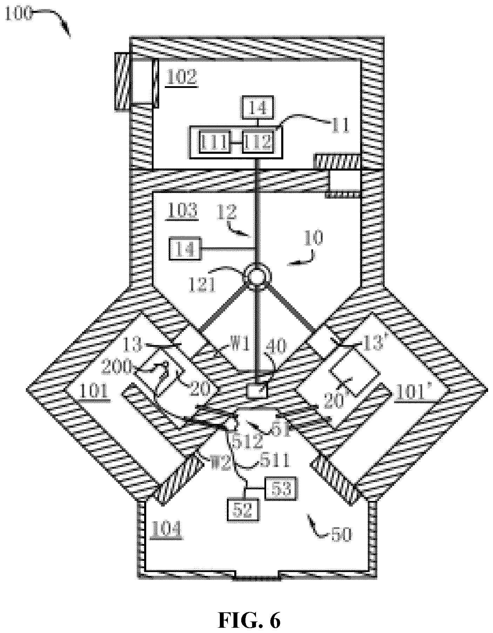

With reference to FIG. 6, the BNCT system 100 is entirely accommodated in a building made of concrete. Specifically, the BNCT system 100 includes a (first) irradiation chamber 101, an accelerator chamber 102 and a beam transmission chamber 103. The irradiated body 200 on the treatment table 20 is subjected to irradiation treatment by the neutron beam N in the irradiation chamber 101. The accelerator chamber 102 at least partially accommodates the charged particle beam generation part 11 (such as the ion source 111, the accelerator 112). The beam transmission chamber 103 at least partially accommodates the beam transmission part 12, and the neutron beam generation part 13 is at least partially accommodated in a partition wall W1 between the irradiation chamber 101 and the beam transmission chamber 103. The auxiliary device 14 is at least partially arranged in the accelerator chamber 102 or the beam transmission chamber 103.

The BNCT system 100 may further include a second irradiation chamber 101', the beam generation device 10 further includes a second neutron beam generation part 13' corresponding to the second irradiation chamber 101', and the beam transmission part 12 includes a beam direction switching assembly 121. With the beam direction switching assembly 121, the beam transmission part 12 may selectively transmit the charged particle beam P generated by the charged particle beam generation part 11 to the first neutron beam generation part 13 or the second neutron beam generation part 13', to emit a beam into the first irradiation chamber 101 or the second irradiation chamber 101'. It should be understood that the neutron beam N irradiated into the second irradiation chamber 101' may be used for irradiation treatment of another irradiated body on the treatment table 20' in the second irradiation chamber 101' by the neutron beam N, and may also be used for sample detection or the like, which is not limited in the invention.

It should be understood that the beam generation device 10 may also have other configurations. For example, when there is a third irradiation chamber, a third neutron beam generation part may be added to correspond to the third irradiation chamber, and the number of neutron beam generation parts corresponds to the number of irradiation chambers, which is not specifically limited in the embodiment of the invention. A charged particle beam generation part is provided to transmit a charged particle beam to each neutron beam generation part, so that the system cost may be effectively reduced. It may be understood that the beam generation device may also include multiple charged particle beam generation parts, to transmit charged particle beams to each neutron beam generation part, and multiple neutron beams may be simultaneously generated in multiple irradiation chambers, to perform irradiation.

In an embodiment of the invention, the beam direction switching assembly 121 includes a deflection magnet (not shown in the figure) deflecting the direction of the charged particle beam P. for example, when a deflection magnet corresponding to the first irradiation chamber 101 is turned on, the beam is introduced into the first irradiation chamber 101, which is not specifically limited in the invention. The BNCT system 100 may also include a beam collector 40 which collects a beam when the beam is not required, or performs an output confirmation of the charged particle beam P before treatment, or the like, and the beam direction switching assembly 121 may allow the charged particle beam P to separate from its normal track and guide the charged particle beam P to the beam collector.

The BNCT system 100 may also include a preparation chamber (not shown in the figure), a control chamber 104, and other spaces (not shown in the figure) for adjuvant therapy. Each irradiation chamber may be provided with a preparation chamber, to fix the irradiated body to the treatment table before irradiation treatment, simulate positioning of the irradiated body, simulate a treatment plan, or the like. The control chamber 104 is configured to control the accelerator, the beam transmission part, the treatment table, or the like, to control and manage the whole irradiation process, and a manager may also monitor multiple irradiation chambers simultaneously in the control chamber. Only one configuration of the control chamber is shown in the figure. It may be understood that the control chamber may also have other configurations.

Since continuous administration is required during BNCT, the BNCT system 100 also includes a medicament injection device 50 configured to inject a boron-containing (B-10) drug into the irradiated body 200 during the irradiation treatment. The medicament inj ection device 50 includes a medicament passage assembly 51 arranged between the medicament control chamber (in the embodiment, it is the control chamber 104) and the irradiation chamber 101. The medicament passage assembly 51 includes a medicament passage member 511 configured to inject the boron-containing (B-10) drug and an accommodation member 512 configured to at least partially accommodate the medicament passage member 511. The irradiation chamber 101 has a partition wall W2 spaced apart from the medicament control chamber, the accommodation member 512 is arranged in the partition wall W2 and forms a passage for the medicament passage member 511 to pass through the partition wall W2, and the accommodation member 512 may further support the medicament passage member 511. In the embodiment, the accommodation member 512 is fixedly arranged in the partition wall W2, for example, mounted in an interference manner. It may be understood that the accommodation member 512 may also be arranged in other ways. On one hand, the accommodation member 512 facilitates passage of the medicament passage member 511, and on the other hand, the accommodation member 512 separates the concrete wall, preventing dust or the like from polluting the medicament passage member 511. Only the device for injecting a boron drug into the irradiated body 200 in the first irradiation chamber 101 is shown in the figure. It may be understood that the same medicament injection device 50 may also be used to inject boron drugs into irradiated bodies in other irradiation chambers respectively.

The medicament injection device 50 may further include a medicament accommodation mechanism 52 and a medicament control mechanism 53, and the medicament accommodation mechanism 52 and the medicament control mechanism 53 may be arranged in the medicament control chamber and control injection of the boron-containing (B-10) drug of the irradiated body 200 in the medicament control chamber, so that neutron radiation rays in the irradiation chamber 101 are prevented from affecting the medicament accommodation mechanism 52 and the medicament control mechanism 53, for example, electronic components in the medicament control mechanism 53 cannot operate normally or react with the boron-containing drug accommodated in the medicament accommodation mechanism 52. The medicament passage member 511 is connected to the medicament accommodation mechanism 52 and injects the boron-containing (B-10) drug into the irradiated body 200 through the medicament control mechanism 53. The medicament accommodation mechanism 52 may be an infusion bag, or an infusion bottle, or the like. The medicament control mechanism 53 may be connected to the medicament passage member 511 and control flow of the boron-containing (B-10) drug in the medicament passage member 511, for example, the medicament control mechanism 53 uses a pump to provide power for flow of liquid (the boron-containing (B-10) drug), may also control a flow rate, and may also have functions of detection, alarm, or the like. The medicament passage member 511 may be a disposable infusion pipe or the like, which includes for example a needle inserted into the irradiated body, a needle protection sleeve, a hose, a joint connected to the medicament accommodation mechanism 52, or the like. The medicament passage member 511 may also be at least partially made of a neutron shielding material, for example, the needle and a part of the hose arranged in the irradiation chamber 101 may be made of a neutron shielding material, which may reduce influence of neutron radiation rays of the irradiation chamber on the boron-containing drug in the medicament passage member 511.

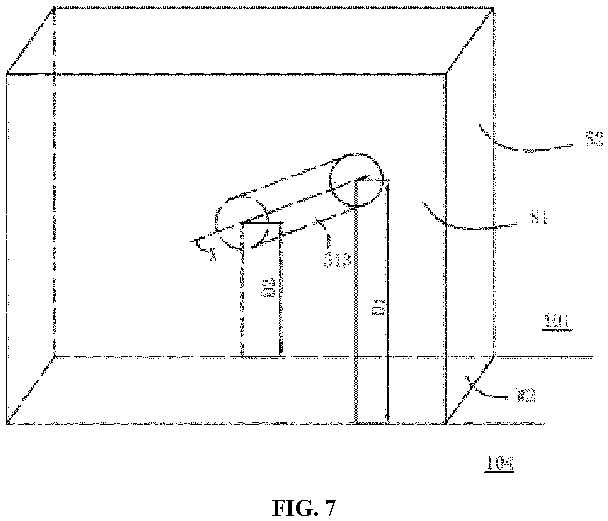

With reference to FIG. 7, in the embodiment, the accommodation member 512 is arranged in a through hole 513 of the partition wall W2 in a thickness direction. A central axis X of the through hole 513 intersects with both the ground and a plane perpendicular to the ground along the thickness direction of the partition wall W2, that is, the through hole 513 passes through the partition wall W2 obliquely in horizontal and vertical directions to reduce radiation leakage, and the central axis X of the through hole 513 is a straight line. It may be understood that the through hole 513 may also be arranged in other ways, for example, the central axis X of the through hole 513 is a broken line or curve, and a cross section of the through hole 513 may be circular, square, or the like. In an embodiment, a distance D1 from center of the through hole 513 located on a first side wall S1 of the partition wall W2 facing the control chamber 104 to the ground is greater than a distance D2 from center of the through hole 513 located on a second side wall S2 of the partition wall W2 facing the irradiation chamber 101 to the ground, for example, the distance from center of the through hole 513 to the ground gradually decreases in a direction from the control chamber 104 to the irradiation chamber 101 along the partition wall W2. In the embodiment, the accommodation member 512 is a tubular member arranged in the through hole 513, an outer wall of the tubular member cooperates with an inner wall of the through hole, an inner wall of the tubular member is not limited in shape. It may be understood that the accommodation member 512 may also be a box body provided with a hole through which the medicament passage member 511 passes, or may be one or more buckles, or the like.

The accommodation member 512 is made ofPVC, and a product after subjecting to neutron irradiation does not have radioactivity or has extremely low radioactivity, thereby reducing the generated secondary radiation. It may be understood that the accommodation member 512 may also be made of another material of which a product after subjecting to neutron irradiation does not have radioactivity or has low radioactivity, or a radioactive isotope generated after subjecting to neutron irradiation has a short half-life period. There may be at least two accommodation members 512 and at least two through holes 513 arranged on each partition wall, so that when one of the accommodation members or one of the through holes is blocked or encounters other problems, the remaining accommodation members or the remaining through holes are used.

Process of injecting the boron-containing (B-10) drug during the irradiation treatment: before starting the irradiation treatment, an appropriate medicament passage member 511 is selected and connected to the medicament accommodation mechanism 52 and the medicament control mechanism 53, and the medicament passage member 511 is placed at an appropriate position in the irradiation chamber 101 by passing through the accommodation member 512; after positioning of the irradiation body 200 in the irradiation chamber 101 is completed and a treatment plan is determined, an operator in the medicament control chamber opens the medicament control mechanism 53, a physician in the irradiation chamber 101 takes the needle protection sleeve off and inserts the needle into the irradiated body 200 or inserts the needle into the irradiated body 200 before positioning of the irradiated body 200, the physician leaves the irradiation chamber 101, and then the operator controls the neutron beam to irradiate the irradiated body and controls injection of the boron-containing (B-10) drug in the control chamber 104. It may be understood that the same medicament injection device 50 (except the accommodation member 512) may also be used to inject the boron-containing (B-10) drug before the irradiation treatment, and the medicament passage member 511 is disconnected before entering the irradiation chamber 101, for example, the needle is pulled out or an indwelling needle is used, and the medicament passage member 511 is reconnected or replaced by a new medicament passage member 511 after entering the irradiation chamber 101, or, control relevant to injection of the boron-containing (B-10) drug before the irradiation treatment or injection of the boron-containing (B-10) drug during the irradiation treatment may also be performed in the preparation chamber, and at this time, the preparation chamber is used as the medicament control chamber. It may be understood that the medicament injection device 50 may also be applied to other types of neutron capture therapy systems, and the boron-containing (B-10) drug may also be replaced by other medicament.

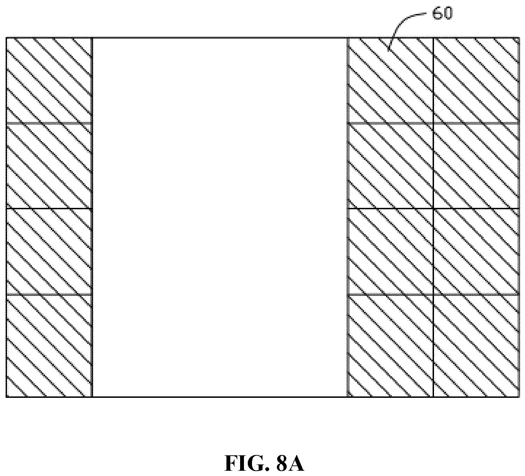

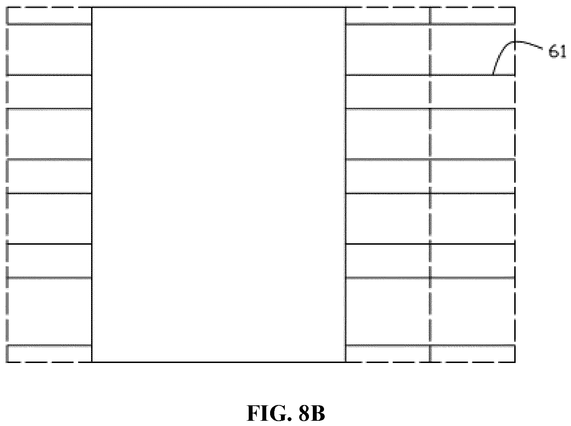

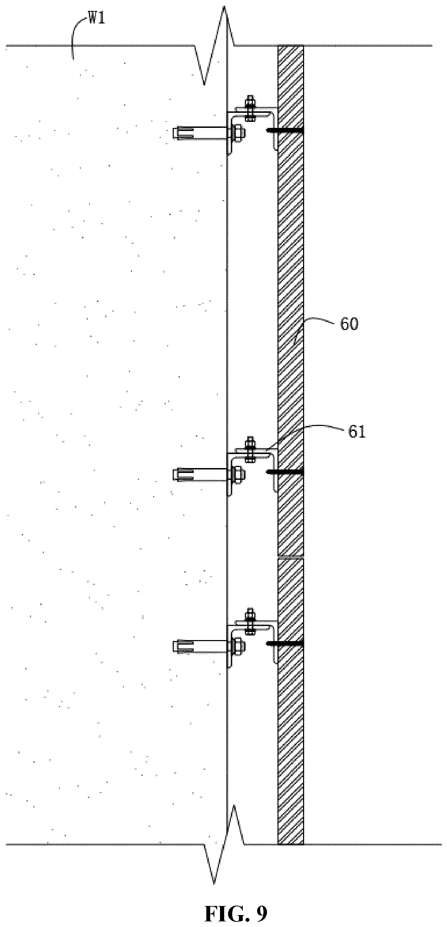

Since a large number of neutrons are generated in a neutron capture therapy process, especially in vicinity of the target T where the neutrons are generated, neutron leakage needs to be avoided as much as possible. In an embodiment, concrete forming at least a part of space (e.g., the beam transmission chamber 103, the irradiation chamber 101, 101') is a concrete added with a neutron shielding material, such as a boron-containing barite concrete, to form a neutron shielding space. In another embodiment, a neutron shielding plate 60, such as a boron-containing PE plate, is arranged on a surface of indoor concrete (e.g., walls or floors or ceilings of the beam transmission chamber 103 and the irradiation chamber 101, 101') to form a neutron shielding space. It may be understood that the neutron shielding plate 60 may be tightly attached to the surface of the concrete, or may be spaced apart from the surface of the concrete by a predetermined distance; the neutron shielding plate may be arranged on the whole surface of the concrete wall, or may be arranged on a partial area of the concrete wall only, for example, the neutron shielding plate is arranged on a surface of floor in a central area of the irradiation chamber, and is not arranged on a surface of floor in an inlet area of the irradiation chamber, and the two areas are connected by a ramp to form a height difference. The neutron shielding plate 60 is arranged on the surface of the concrete through a support assembly 61, as shown in FIG. 8A and FIG. 8B which show layouts of the neutron shielding plate 60 and the support assembly 61 arranged at a side, facing the beam transmission chamber 103, of the partition wall W1 between the irradiation chamber 101 and the beam transmission chamber 103. FIG. 9 shows manners of fixing the neutron shielding plate 60 and the support assembly 61. The neutron shielding plate 60 is formed by a combination of several pieces. Strip-shaped support assemblies 61 are arranged on concrete of the partition wall W1 by expansion bolts at preset intervals. Each piece of the neutron shielding plate 60 is sequentially fixed to a corresponding position on the support assembly 61 by screws, that is, one side of the support assembly 61 is connected to the concrete, and the other side of the support assembly 61 is connected to the neutron shielding plate 60. In the embodiment, the support assembly 61 includes two L-shaped plates connected by bolts. It may be understood that the support assembly 61 may also be arranged and fixed in other ways, for example, the support assembly 61 is at least partially made of a profile, or the neutron shielding plate 60 may be directly fixed on the surface of the concrete; and the neutron shielding plate 60 may also be arranged on a sidewall of an accommodation groove of the partition wall W1 accommodating the neutron beam generation part 13.

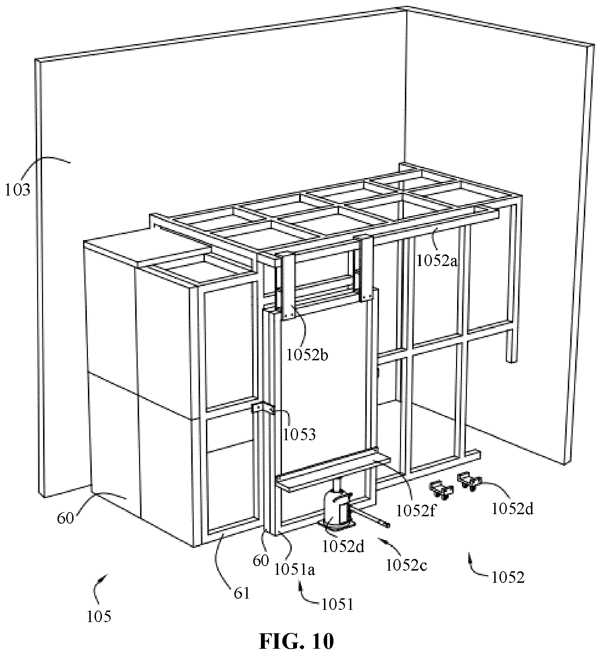

In order to reduce radiation damage and radiation pollution of neutrons to other indoor devices such as the auxiliary device 14 in the neutron capture therapy process, the neutron shielding plate 60 may be arranged around the auxiliary device 14 to form a shielding space. As shown in FIG. 10, in an embodiment, an auxiliary device compartment 105 is provided in the beam transmission chamber 103 to accommodate or surround the auxiliary device 14, or the like. The auxiliary device compartment 105 is at least partially made of the support assembly 61 and the neutron shielding plate 60 fixed on the support assembly 61 (only a part of the neutron shielding plate is shown in the figure). In the embodiment, the auxiliary device compartment 105 is arranged at a corner of the beam transmission chamber 103 and shares a part of wall and floor of the beam transmission chamber 103. The support assembly 61 and the neutron shielding plate 60 fixed on the support assembly 61 and a part of wall and floor of the beam transmission chamber 103 jointly form a space accommodating and surrounding the auxiliary device 14. That is, the neutron shielding plate 60 fixed on the support assembly 61 forms three surfaces of an accommodation space of a cube, and a part of wall and floor of the beam transmission chamber 103 form another three surfaces of the accommodation space of the cube. The auxiliary device compartment 105 may further have a door 1051 and a mobile mechanism 1052 thereof, the mobile mechanism 1052 is configured to open the door 1051 to allow an operator to enter interior of the auxiliary device compartment 105 when the device is examined and repaired, and the mobile mechanism 1052 includes a guide rail 1052a and a sliding rod 1052b, the door 1051 may slide along the guide rail 1052a in a horizontal direction through the sliding rod 1052b. In the embodiment, the door 1051 is made of a door support assembly 1051a and the neutron shielding plate 60 fixed on the door support assembly 1051a, the sliding rod 1052b is fixedly connected to the door support assembly 1051a, for example, arranged at a top end of the door 1051, and the guide rail 1052a is fixedly connected to the support assembly 61 of the auxiliary device compartment 105. It may be understood that the mobile mechanism 1052 may also have other configurations, for example, the door is rotatable. The mobile mechanism 1052 may further include a lifting assembly 1052c and a pulley 1052d, the lifting assembly 1052c is configured to lift the door 1051 in a vertical direction to place the pulley 1052d at the bottom of the door 1051, so that the door 1051 may slide in the horizontal direction by means of the pulley 1052d. In the embodiment, the lifting assembly 1052c is made of a jack 1052e and a connection plate 1052f fixed on the door support assembly 1051a, and the jack 1052e acts on the connection plate 1052f, so that the door 1051 slides along the guide rail 1052a in the vertical direction through the sliding rod 1052b, so as to lift the door 1051 in the vertical direction. It may be understood that the lifting assembly 1052c may also have other configurations. The auxiliary device compartment 105 may further include a fixing member 1053 taking effect when the door 1051 is closed, to fix the door 1051 and the auxiliary device compartment 105 together so as to reinforce fixation and prevent rollover. In the embodiment, the fixing member 1053 is made of an L-shaped plate of which two side plates are fixed to the door support assembly 1051a and the support assembly 61 or the neutron shielding plate 60 of the auxiliary device compartment 105 respectively. The auxiliary device compartment 105 may also have an opening 1054 for pipes, cables, or the like to pass through. In the embodiment, the opening 1054 is arranged near a corner of the wall and floor. The support assembly 61 of the auxiliary device compartment 105 and the door support assembly 1051a are made of mutually connected profiles. It may be understood that the auxiliary device compartment 105 may also have other configurations, and auxiliary device compartments may also be provided in other spaces.

The neutron shielding plate 60 is a boron-containing PE plate, and material of each of the support assembly 61, the door support assembly 1051a, the guide rail 1052a, the sliding rod 1052b and the fixing member 1053 is an aluminum alloy. It may be understood that material of the neutron shielding plate 60 may also be another neutron shielding material, different thicknesses may be achieved at different positions according to requirements, and the surface may have other decorations or grooves to mount other elements; and the aluminum alloy may be replaced by another material which has a certain strength and of which a product after subjecting to neutron irradiation does not have radioactivity or has low radioactivity, or a radioactive isotope generated after subjecting to neutron irradiation has a short half-life period, such as a carbon fiber composite material or a glass fiber composite material.

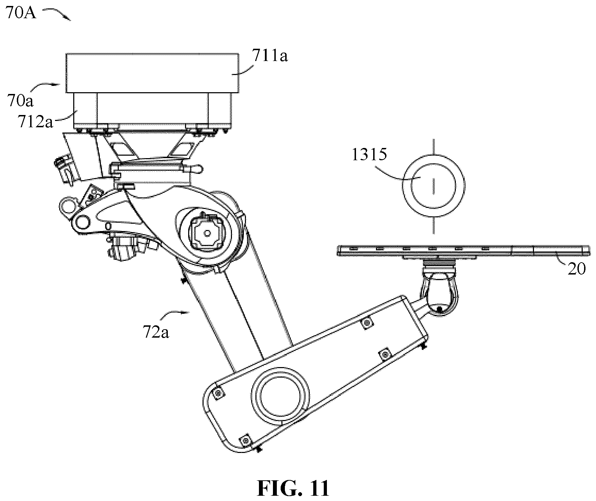

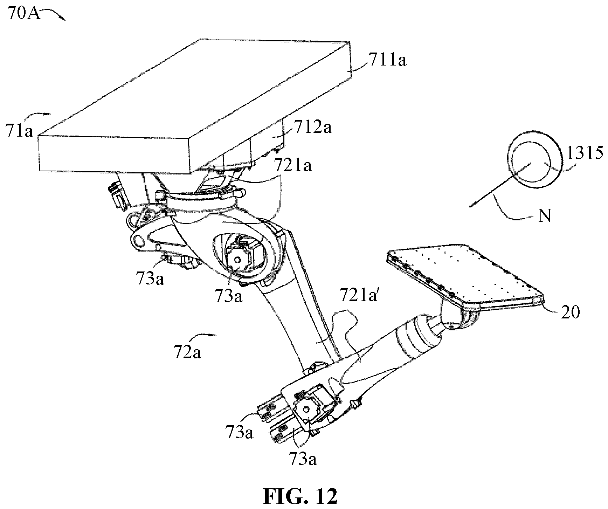

With reference to FIG. 11 to FIG. 13, the irradiation chamber 101, 101' may also be provided with a treatment table positioning device 70A and a shielding device 70B of the treatment table positioning device. The treatment table positioning device 70A includes a linear shaft 71a and a robotic arm 72a, and the robotic arm 72a is arranged between the linear shaft 71a and the treatment table 20 to support and position the treatment table 20, connects the treatment table 20 to the linear shaft 71a, and enables the treatment table 20 and the robotic arm 72a to translate along the linear shaft 71a together. In the embodiment, the linear shaft 71a is mounted to ceiling of the irradiation chamber, and the robotic arm 72a entirely extends toward floor of the irradiation chamber. It may be understood that the linear shaft 71a may also be mounted to other surfaces, such as wall or floor; the linear shaft 71a is configured to be fixed to a sliding rail 711a of the ceiling and a support seat 712a connected to the robotic arm 72a and sliding along the sliding rail 711a. It may be understood that there may also be other configurations. The linear shaft 71a is directly fixed on the ceiling, a linear shaft fixing mechanism such as a steel structure gantry, is not provided additionally, which reduces amount of steel in the irradiation chamber and reduces secondary radiation due to the fixing mechanism activated by neutrons. The robotic arm 72a is a multi-axis robotic arm connecting the support seat 712a to the treatment table 20, and includes multiple arm parts 721a (721a').

Since the support seat 712a connected to the robotic arm 72a slides along the sliding rail 711a, the neutron shielding plate 60 arranged on the ceiling or another fixing surface needs to be reserved with a sliding space, which induces exposure of the sliding rail and radiation leakage. Therefore, the shielding device 70B includes a sliding rail covering member 71b, and the sliding rail covering member 71b moves together with the support seat 712a and always covers an exposed part of the sliding rail 71 1a. The shielding device 70B further includes a robotic arm sheath 72b surrounding at least one arm part 721a (721a') of the robotic arm 72a, and material of the robotic arm sheath 72b is at least partially a neutron shielding material, to prevent the arm part and metal components, electronic components, or the like arranged in mechanisms of the arm part from being irradiated by neutrons to become failure or damaged, such as a boron-containing glass fiber composite material. It may be understood that other shielding materials may also be used.

The treatment table positioning device 70A may further include a driving mechanism 73a, and the irradiation chamber 101, 101' or the control chamber 104 may also be provided with a treatment table control device 70C connected to the driving mechanism 73a and controlling movement of the linear shaft 71a and the robotic arm 72a by controlling the driving mechanism 73a. Position information of the linear shaft 71a and the robotic arm 72a may also be fed back to the treatment table control device 70C, and the driving mechanism 73a may be arranged on the linear shaft 71a or the robotic arm 72a, such as the support seat 712a or at least one arm part 721a.

The treatment table positioning device 70A may further include an anti-collision protection mechanism 74a, and the anti-collision protection mechanism 74a includes a sensor 741a arranged on the robotic arm sheath 72b, a sensor control assembly 742a and an HMI 743a. It may be understood that the sensor 741a may also be arranged between the robotic arm sheath 72b and the robotic arm 72a. When an edge of the robotic arm 72a or the robotic arm sheath 72b contacts other objects, or when other objects reach in a range set by the sensor 741a, the sensor 741a is triggered to transmit a signal, and the signal transmitted by the sensor 741a is transmitted to the sensor control assembly 742a and displayed on the HMI 743a. The sensor control assembly 742a transmits the received signal to the treatment table control device 70C to perform corresponding control, for example, the treatment table control device 70C controls the driving mechanism 73a to stop driving movement of the linear shaft 71a and the robotic arm 72a, that is, controls the treatment table 20 to stop moving. It may be understood that the sensor control assembly may also perform corresponding control according to the received signal; an operator may also manually control the driving mechanism to stop driving according to display of the HMI; or, the treatment table may not be controlled to stop moving, instead, other safe operations, such as inverse movement before collision, are performed. The sensor 741a may be a mechanical sensor, a photoelectric sensor, a radar sensor, an ultrasonic sensor, a laser range finder, or the like, and may also be arranged at other positions.

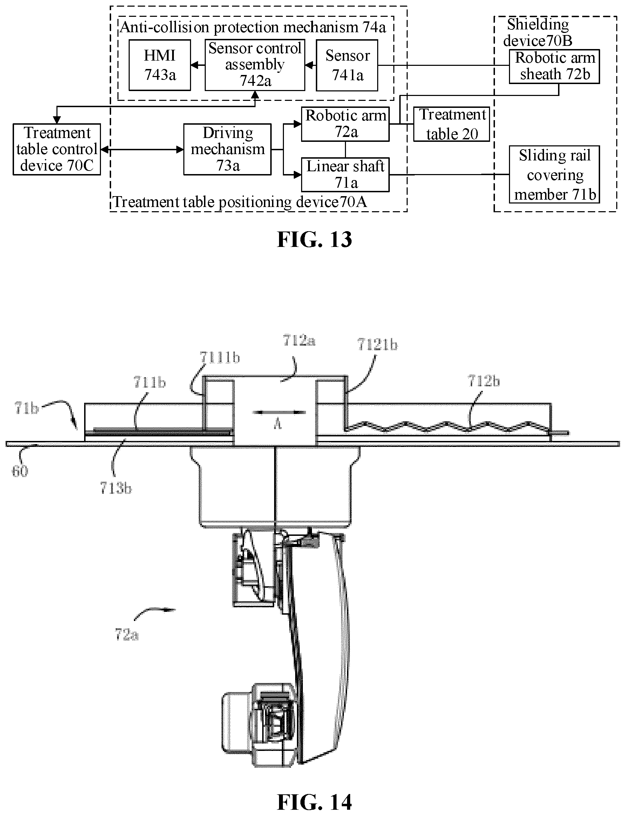

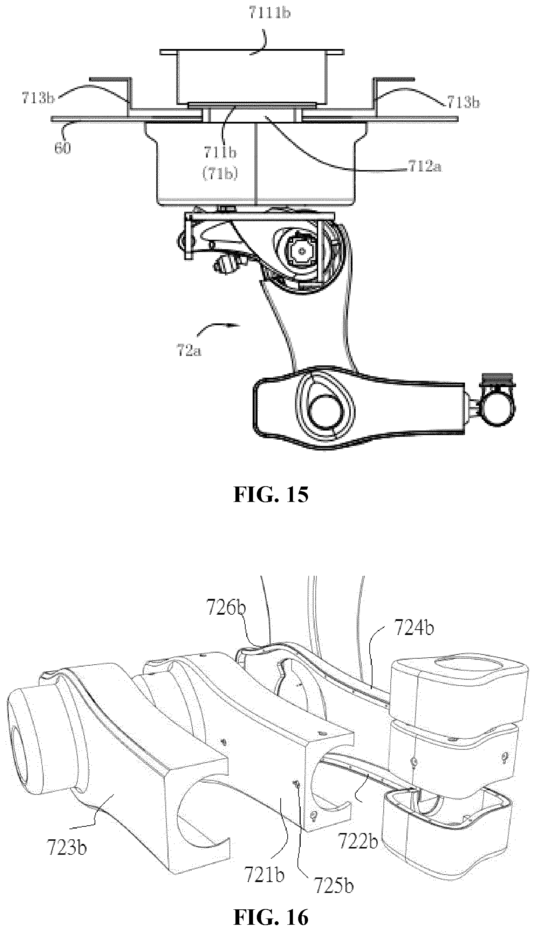

The linear shaft 71a and the driving mechanism 73a thereof may be mounted to a fixing surface of the irradiation chamber 101, 101' by fixing members or support members (not shown in the figure), and each of the fixing member and the support member may be made of an aluminum profile, for example, the sliding rail 711a is fixed on the ceiling by the fixing members, the driving mechanism 73a of the linear shaft 71a and the support seat 712a are fixed or supported on the ceiling by the support members, and the sliding rail covering member 71b is arranged between the neutron shielding plate 60 on a fixing surface of the linear shaft 71a and the support seat 712a. As shown in FIG. 14 and FIG. 15, in an embodiment, the sliding rail covering member 71b includes a first part 711b and a second part 712b, and each of the first part 711b and the second part 712b includes flat plates connected in sequence and is supported by a support member 713b of the sliding rail covering member. One end, close to the support seat 712a along a sliding direction A of the support seat 712a, of each of the first part 711b and the second part 712b is fixedly connected to the support seat 712a through connection plates 71 11b, 7121b, and the other end of each of the first part 711b and the second part 712b is fixedly connected to the support member 713b. It may be understood that the fixed connection may be screw connection, bonding, or the like; the flat plates of the first part 711b and the second part 712b are slidably connected in sequence (such as the first part 711b shown in a left side of FIG. 14) or pivotally connected in sequence (such as the second part 712b as shown in a right side of FIG. 14). It may be understood that the flat plates may also be connected in other ways, different connection manners are shown in the figure only, and the same or different connection manners may be selected for the first part 711b and the second part 712b according to requirements. The support member 713b may be connected to the fixing member or the support member of the linear shaft 71a and the driving mechanism 73a thereof so as to be fixed, or may be directly fixed to the fixing surface. The support member 713b is made of an aluminum alloy, material of the sliding rail covering member 71b includes a boron-containing PE or another neutron shielding material, and the neutron shielding plate 60 covers the support member 713b and shields the linear shaft 71a, the driving mechanism 73a of the linear shaft 71a and a mounting part thereof (except a portion where the support seat 712a passes through the neutron shielding plate 60) together with the sliding rail covering member 71b. It may be understood that the aluminum alloy may be replaced by another material which has a certain strength and of which a product after subjecting to neutron irradiation does not have radioactivity or has low radioactivity, or a radioactive isotope generated after subjecting to neutron irradiation has a short half-life period; the support member 713b may also be made of a neutron shielding material, and at this time, the neutron shielding plate 60 may not cover the support member 713b, instead, matches the support member 713b, and the neutron shielding plate 60, the support member 713b and the sliding rail covering member 71b together shield the linear shaft 71a, the driving mechanism 73a of the linear shaft 71a and a mounting part thereof (except a portion where the support seat 712a passes through the neutron shielding plate 60). During movement of the support seat 712a along the sliding rail 711a, the first part 711b and the second part 712b of the sliding rail covering member 71b extend and retract, thereby reducing neutron leakage throughout the movement process.

With reference to FIG. 16, in the embodiment, the robotic arm sheath 72b surrounding the arm part 721a includes a first housing 721b and a second housing 722b, the first housing 721b and the second housing 722b are fixedly connected together and surround the arm part 721a and the driving mechanism 73a (such as a motor, a circuit board, or the like) or a control mechanism (such as components of the sensor control assembly 742a or the treatment table control device 70C) arranged on the arm part 721a. Material of each of the first housing 721b and the second housing 722b is a boron-containing glass fiber composite material, the glass fiber composite material has a certain strength, of which a product after subjecting to neutron irradiation does not have radioactivity or has low radioactivity, to prevent generation of secondary radiation, and boron may absorb neutrons, to prevent the arm part and metal components, electronic components, or the like arranged in the driving mechanism or the control mechanism of the arm part from being irradiated by neutrons to become failure or damaged. It may be understood that the material of each of the first housing and the second housing may also be another neutron shielding material with a certain strength.

In the embodiment, the robotic arm sheath 72b' surrounding the arm part 721a' further includes a third housing 723b and a fourth housing 724b, besides the first housing 721b and the second housing 722b. The third housing 723b and the fourth housing 724b are fixedly connected together and surround the first housing 721b and the second housing 722b, and the sensor 741a is arranged between the first housing 721b and the third housing 723b and between the second housing 722b and the fourth housing 724b. There may be multiple sensors 741a distributed around the arm part 721a. The first housing 721b and the second housing 722b are provided with an accommodation cavity 725b accommodating the sensor 741a, and the sensor 741a is arranged in the accommodation cavity 725b and mounted between the first housing 721b and the third housing 723b and between the second housing 722b and the fourth housing 724b in an interference manner. Specifically, a gap 726b is provided between the first housing 721b and the third housing 723b and between the second housing 722b and the fourth housing 724b, and configured to mount the sensor 741a therein. A power supply cable, a communication cable, or the like of the sensor 741a may pass through the gap 726b to be connected to the sensor control assembly 742a. Or, the third housing 723b and the fourth housing 724b may be provided with a through hole 727b (not shown in the figure) at a position corresponding to the sensor 741a, and the through hole 727b is used for a power supply cable, a communication cable, or the like of the sensor 741a to pass through. It may be understood that the sensor 741a may also be mounted in other ways. In the embodiment, the sensor 741a is a pressure sensor, converts pressure acting on the third housing 723b and the fourth housing 724b into a pressure signal and transmits the pressure signal to the sensor control assembly 742a, and displays a numerical value thereof in the HMI 743a. When the pressure signal received by the sensor 741a exceeds a preset value, the pressure signal exceeding the preset value is preferentially transmitted to the sensor control assembly 742a and is displayed in the HMI 743a in an alarm manner, for example, by light or a sound alarm; the sensor control assembly 742a transmits the signal to the treatment table control device 70C to control the linear shaft 71a and the robotic arm 72a to stop moving, or an operator may manually operate to stop movement of the linear shaft 71a and the robotic arm 72a.

Material of each of the third housing 723b and the fourth housing 724b is a glass fiber resin composite material with a certain strength, of which a product after subjecting to neutron irradiation does not have radioactivity or has low radioactivity, to prevent generation of secondary radiation. It may be understood that another material which has a certain strength and of which a product after subjecting to neutron irradiation does not have radioactivity or has low radioactivity, or a radioactive isotope generated after subjecting to neutron irradiation has a short half-life period, may also be used. It may be understood that material of each of the third housing and the fourth housing may also be replaced by a boron-containing glass fiber composite material, that is, a housing at the outermost layer of the robotic arm sheath 72b is made of a material capable of absorbing neutrons, to prevent metal components, electronic components, or the like arranged in the driving mechanism or the control mechanism of the arm part from being irradiated by neutrons to become failure or damaged, and materials of the first housing and the second housing are not limited. A housing of the sensor 741a is made of an aluminum alloy, avoiding usage of a traditional steel material which generates a radioactive isotope with a long half-life period after subjecting to neutron irradiation, such as cobalt sixty, thereby generating secondary radiation. It may be understood that the aluminum alloy may be replaced by another material which has a certain strength and of which a product after subjecting to neutron irradiation does not have radioactivity or has low radioactivity, or a radioactive isotope generated after subjecting to neutron irradiation has a short half-life period. It may be understood that the sensor 741a may also be arranged between the first housing 721b and the third housing 723b or between the second housing 722b and the fourth housing 724b only.

Manners of fixedly connecting the first housing 721b to the second housing 722b and fixedly connecting the third housing 723b to the fourth housing 724b may be screw connection, welding, or the like. The connection member is made of an aluminum alloy with a certain strength, and a radioactive isotope generated after aluminum subjecting to neutron activation has a short half-life period. The aluminum alloy may be replaced by another material which has a certain strength and of which a product after subjecting to neutron irradiation does not have radioactivity or has low radioactivity, or a radioactive isotope generated after subjecting to neutron irradiation has a short half-life period.

In the embodiment, the third housing 723b, the fourth housing 724b and the sensor 741a are arranged in the arm part 721a' having a larger movement range, and only the first housing 721b and the second housing 722b are arranged in the arm part 721a having a smaller movement range. It may be understood that the third housing 723b, the fourth housing 724b and the sensor 741a may also be arranged in all the arm parts of the robotic arm 72a; an arm part without the driving mechanism 73a may not be provided with a robotic arm sheath 72b, and at this time, the arm part is made of a material which has a certain strength and of which a product after subjecting to neutron irradiation does not have radioactivity or has low radioactivity, or a radioactive isotope generated after subjecting to neutron irradiation has a short half-life period, such as an aluminum alloy, or may be made of a neutron shielding material.

It may be understood that the treatment table positioning device 70A may not include the linear shaft, and at this time, the shielding device 70B does not include the sliding rail covering member 71b either, and the preparation chamber may also be provided with the same treatment table 20, the treatment table positioning device 70A and the shielding device 70B of the treatment table positioning device as in the irradiation chamber 101, 101'.

It may be understood that radiation shielding devices may also be provided for other alarm, supervisory, monitoring devices, or the like.

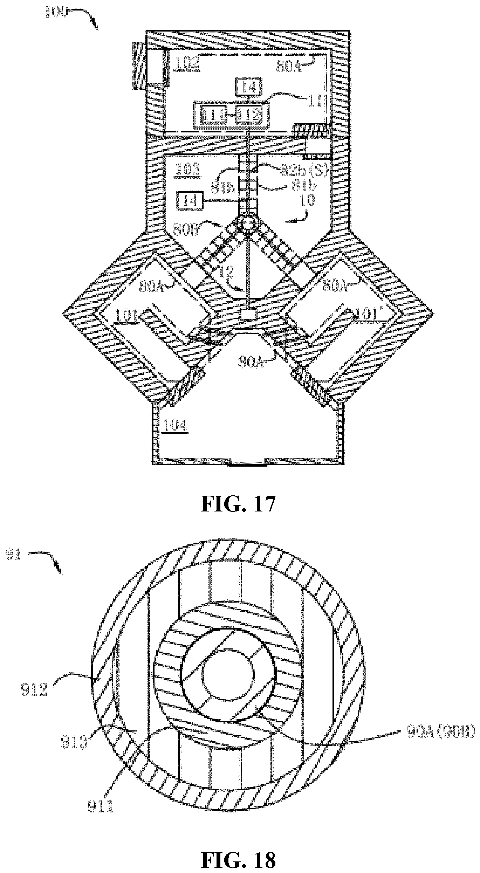

In order to achieve operation of each device of the system, control during the treatment needs to be provided with a power supply cable, a communication cable and a control cable with a reasonable arrangement. As shown in FIG. 17, a threading pipe 80A is arranged in the irradiation chamber 101, the control chamber 104 and the accelerator chamber 102, the threading pipe 80A is used for the cable to pass through and supporting the cable, and the threading pipe 80A extends along an extension direction of the cable and is at least partially closed in a circumferential direction around the extension direction of the cable, and cross-sectional shape of the threading pipe 80A in a direction perpendicular to the extension direction of the cable may be circular, polygonal, V -shaped, < -shaped, 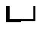 -shaped, [-shaped, or the like, and the threading pipe 80A is fixed on a wall or floor or ceiling by connectors (such as bolts). In the embodiment, the threading pipe 80A is arranged in the irradiation chamber 101, the control chamber 104 and the accelerator chamber 102 along a corner of the ceiling and wall. It may be understood that the threading pipe 80A may also be arranged at other positions or other spaces, and size of the threading pipe 80A may be designed according to the number of accommodated cables. A support frame 80B is arranged in the accelerator chamber 102 and the beam transmission chamber 103. Since the accelerator 112, the beam transmission part 12, the auxiliary device 14, or the like have many power supply, communication, control cables and liquid (such as a cooling medium) or gas (such as an insulation gas) pipes, the support frame 80B is provide to bear and guide them. The support frame 80B has a bearing surface S supporting the cable or pipe, and the support frame 80B is fixed to the ground or ceiling or other objects in a manner that the bearing surface S is parallel to the ground, or fixed on the wall in a manner that the bearing surface S is perpendicular to the ground, and the support frame 80B may also be arranged in other spaces according to requirements. Only the support frame 80B arranged along the beam transmission part 12 in the beam transmission chamber 103 is shown in the figure, the support frame 80B is fixed to the ground in a manner that the bearing surface S is parallel to the ground, and the support frame 80B is made of side plates 81b and transverse plates 82b connected between the side plates 81b at predetermined intervals, and the transverse plates 82b form the bearing surface S. Material of each of the threading pipe 80A and the support frame 80B is an aluminum alloy, and it may be understood that the aluminum alloy may be replaced by another material which has a certain strength and of which a product after subjecting to neutron irradiation does not have radioactivity or has low radioactivity, or a radioactive isotope generated after subjecting to neutron irradiation has a short half-life period, for example, the material is composed of at least one of C, H, O, N, Si, Al, Mg, Li, B, Mn, Cu, Zn, S, Ca or Ti element, in 90% (percentage in terms of weight) or more thereof.

With respect to normal operation and safety requirements of the system, a tubular member 90A (such as a ventilation pipe, a firefighting pipe, or the like, for gas and liquid to pass through) and a rod-shaped member 90B (support rod, screw rod and other fixing rods required for fixedly mounting various devices) are also provided indoors and are usually made of steel materials, which may generate a radioactive isotope with a long half-life period after subjecting to neutron irradiation, such as cobalt sixty, thereby generating secondary radiation. In order to reduce radiation damage and radiation pollution to pipes and fixing rods, the tubular member 90A (including the above cooling medium and an insulation gas pipe) or the rod-shaped member 90B may be made of a material of which a product after subjecting to neutron irradiation does not have radioactivity or has low radioactivity, or a radioactive isotope generated after subjecting to neutron irradiation has a short half-life period (for example, the material is composed of at least one of C, H, O, N, Si, Al, Mg, Li, B, Mn, Cu, Zn, S, Ca or Ti element, in 90% (percentage in terms of weight) or more thereof, including an aluminum alloy, plastic, or rubber, or the like), or an annular shielding device 91 is arranged on a periphery of the tubular member 90A or the rod-shaped member 90B. As shown in FIG. 18, in an embodiment, the annular shielding device 91 includes an inner sleeve 911, an outer sleeve 912, and a shielding material 913 arranged between the inner sleeve 911 and the outer sleeve 912. Each of the inner sleeve 911 and the outer sleeve 912 is a tubular component made of PVC, and cross-sectional shape of each of the inner sleeve 911 and the outer sleeve 912 may be set according to specific requirements. It may be understood that each of the inner sleeve 911 and the outer sleeve 912 may also be made of another material of which a product after subjecting to neutron irradiation does not have radioactivity or has low radioactivity, or a radioactive isotope generated after subjecting to neutron irradiation has a short half-life period, for example, material of each of the inner sleeve 911 and the outer sleeve 912 is composed of at least one of C, H, O, N, Si, Al, Mg, Li, B, Mn, Cu, Zn, S, Ca or Ti element, in 90% (percentage in terms of weight) or more thereof. The outer sleeve 912 may also be used as a neutron retarder, and the retarded neutron may be better absorbed by the shielding material 913. The shielding material 913 is composed of a neutron shielding material, such as a boron-containing resin. In an embodiment, a liquid-like boron-containing resin is filled between the inner sleeve 911 and the outer sleeve 912 made of PVC, and the boron-containing resin is solidified to form the entirety of the annular shielding device 91, and then the annular shielding device 91 is cut into two parts along a plane where a central axis thereof is located, to wrap the cable, the tubular member 90A or the rod-shaped member 90B from two sides, and then the two parts are fixedly connected by gluing, bundling, or other manners. It may be understood that the shielding material 913 may also include another neutron shielding material or may be arranged between the inner sleeve 911 and the outer sleeve 912 in other ways. The annular shielding device 91 may also be arranged on the periphery of the tubular member 90A or the rod-shaped member 90B in other ways, for example, the tubular member 90A or the rod-shaped member 90B is inserted into the inner sleeve 911 of the annular shielding device 91 before the tubular member 90A or the rod-shaped member 90B is mounted. It may be understood that the annular shielding device 91 may also be arranged on a periphery of the cable, to further reduce secondary radiation generated after the cable subjecting to neutron irradiation.

While the illustrative specific implementations of the invention have been described as above, so that those skilled in the art understand the invention, it should be apparent that the invention is not limited to the scope of the specific implementations, various changes are apparent for those of ordinary skill in the art and fall within the scope of protection of the invention, as long as these changes fall within the spirit and scope of the invention as defined and determined by the appended claims.

## Claims

1. A neutron capture therapy system, **characterized in that** the neutron capture therapy system comprises a charged particle beam generation part, a beam transmission part and a neutron beam generation part, the charged particle beam generation part comprises an ion source configured to generate charged particles and an accelerator configured to accelerate the charged particles generated by the ion source to obtain a charged particle beam with a required energy, the neutron beam generation part comprises a target, a beam shaping body and a collimator, the target is arranged between the beam transmission part and the beam shaping body, the charged particle beam generated by the accelerator is irradiated onto the target through the beam transmission part and acts with the target to generate neutrons, and the generated neutrons sequentially pass through the beam shaping body and the collimator to form a therapeutic neutron beam, the neutron capture therapy system is entirely accommodated in a building made of concrete and comprises an irradiation chamber, an accelerator chamber and a beam transmission chamber, an irradiated body injected with a medicament is subjected to irradiation treatment by the therapeutic neutron beam in the irradiation chamber, the accelerator chamber at least partially accommodates the charged particle beam generation part, the beam transmission chamber at least partially accommodates the beam transmission part, and the neutron beam generation part is at least partially accommodated in a partition wall between the irradiation chamber and the beam transmission chamber.

2. The neutron capture therapy system of claim 1, wherein the neutron capture therapy system further comprises: a medicament control chamber; and a medicament injection device configured to inject the medicament into the irradiated body during the irradiation treatment and comprising a medicament passage assembly, a medicament accommodation mechanism and a medicament control mechanism, the medicament passage assembly arranged between the medicament control chamber and the irradiation chamber, and the medicament accommodation mechanism and the medicament control mechanism arranged in the medicament control chamber and controlling injection of the medicament of the irradiated body in the medicament control chamber.

3. The neutron capture therapy system of claim 2, wherein the medicament passage assembly comprises: a medicament passage member configured to inject the medicament; and an accommodation member configured to at least partially accommodate the medicament passage member, arranged in the partition wall and forming a passage for the medicament passage member to pass through the partition wall.

4. The neutron capture therapy system of claim 1, wherein the neutron capture therapy system further comprises a treatment table, a treatment table positioning device, and a shielding device of the treatment table positioning device.

5. The neutron capture therapy system of claim 4, wherein the treatment table positioning device comprises a robotic arm configured to support and position the treatment table and comprising at least one arm part, and the shielding device comprises a robotic arm sheath surrounding the arm part.

6. The neutron capture therapy system of claim 5, wherein the robotic arm sheath is provided with an anti-collision protection mechanism.

7. The neutron capture therapy system of claim 5, wherein the treatment table positioning device further comprises a linear shaft, the robotic arm is arranged between the linear shaft and the treatment table, the linear shaft comprises a sliding rail fixed to the building and a support seat connected to the robotic arm, the support seat drives the treatment table and the robotic arm to slide along the sliding rail together, and the shielding device comprises a sliding rail covering member.

8. The neutron capture therapy system of claim 1, wherein a neutron shielding space is formed in the building and formed in the beam transmission chamber or the irradiation chamber, the concrete is a boron-containing barite concrete, or a neutron shielding plate is arranged on a surface of the concrete to form the neutron shielding space.

9. The neutron capture therapy system of claim 1, wherein the building is internally provided with a cable for operations of the neutron capture therapy system, or a tubular member for gas and liquid to pass through, or a rod-shaped member fixedly mounted in the building, or a support device supporting the cable or the tubular member, and a material of the support device, the tubular member or the rod-shaped member is composed of at least one of C, H, O, N, Si, Al, Mg, Li, B, Mn, Cu, Zn, S, Ca or Ti element, in 90% (percentage in terms of weight) or more thereof, or a periphery of the cable, the tubular member or the rod-shaped member is provided with an annular shielding device comprising an inner sleeve, an outer sleeve, and a shielding material arranged between the inner sleeve and the outer sleeve.

10. The neutron capture therapy system of claim 1, wherein the neutron capture therapy system further comprises an auxiliary device at least partially arranged in the accelerator chamber or the beam transmission chamber and comprising a cooling device, or an insulation gas inflation and recovery device, or an air compression device providing compressed air, or a vacuum pump providing a vacuum environment.

11. The neutron capture therapy system of claim 10, wherein a cooling medium of the cooling device has hardness less than 60 mg/L.

12. The neutron capture therapy system of claim 10, wherein the cooling device is configured to cool the ion source or the accelerator or the target, a cooling medium of the cooling device has hardness less than 17 mg/L, or the cooling medium of the cooling device is deionized water with a conductivity of 0.5-1.5 µS/cm.

13. The neutron capture therapy system of claim 10, wherein the cooling device comprises an external circulation device, an internal circulation device and a heat exchanger, the internal circulation device delivers a cooling medium to a to-be-cooled component of the neutron capture therapy system to absorb heat thereof, then delivers the cooling medium after heat absorption and temperature rise to the heat exchanger, to perform heat exchange with chilled water delivered to the heat exchanger by the external circulation device, and then delivers the cooling medium after temperature dropping to the to-be-cooled component again to absorb heat thereof, and the external circulation device is capable of continuously providing the chilled water to the heat exchanger and recovering the chilled water after heat absorption and temperature rise.

14. The neutron capture therapy system of claim 10, wherein the accelerator comprises an accelerator high-voltage power supply providing an acceleration energy and internally provided with an insulation gas, and the insulation gas inflation and recovery device provides the insulation gas for the accelerator high-voltage power supply or recovers the insulation gas from the accelerator high-voltage power supply.

15. The neutron capture therapy system of claim 14, wherein the insulation gas inflation and recovery device comprises a gas source comprising a container containing the insulation gas, and a storage container connected to the gas source and the accelerator high-voltage power supply respectively.
